# EUROPEAN PATENT APPLICATION

(11) **EP 4 831 200 A1**
(43) Date of publication of application: **28.10.2026**
(21) Application number: 24906453.6
(22) Date of filing: 19.12.2024
(51) Int. Cl.: C07K 16/28, A61K 39/395, A61P 37/00

(54) **ANTIBODY TARGETING CXCR6 AND USE THEREOF**

(30) Priority: 20.12.2023 CN 202311766847
(71) Applicant: Mabwell (Shanghai) Bioscience Co., Ltd., Shanghai 201210 (CN)
(72) Inventor: LI, Zhongfei, Shanghai 201210 (CN); REN, Wei, Shanghai 201210 (CN); YUAN, Weilin, Shanghai 201210 (CN); LIU, Chuang, Shanghai 201210 (CN); GUI, Xun, Shanghai 201210 (CN); LIU, Datao, Shanghai 201210 (CN)
(74) Representative: Bianchetti & Minoja with Trevisan & Cuonzo IPS SRL
(86) International application number: PCT/CN2024/140592
(87) International publication number: WO 2025/130978

(57) **Abstract**

The present invention provides an anti-CXCR6 antibody and the use thereof. The anti-CXCR6 antibody can bind to CXCR6 with high affinity and high specificity, has cross-species binding activity to both human CXCR6 and monkey CXCR6, is a depleting-antibody targeting CXCR6, and has the potential of treating inflammation or immune disorder diseases. Compared with existing CXCR6 antibodies, the anti-CXCR6 antibody involves lower cell non-specific binding activity, lower off-target toxicity and higher safety.

## Description

### Cross-Reference to Related Applications

This patent application claims the priority benefit of Chinese invention patent application No. CN202311766847.6, filed on December 20, 2023, which is incorporated herein by reference in its entirety.

### Technical Field

The present invention relates to the field of antibody drugs. Particularly, the present invention relates to an anti-CXCR6 antibody and a use thereof for the manufacture of a medicament.

### Background Art

CXC-family chemokine receptor 6 (CXCR6), also known as Bonzo, STRL33, and TYMSTR. CXCR6 is expressed on activated T cells, including CD4+ T cells (Th1, Th17 cells), CD8+ T cells, and others. These activated T cells are key drivers of various inflammatory or immune dysregulation diseases, including, but not limited to, autoimmune diseases, graft-versus-host disease (GvHD), and neurodegenerative diseases. For example, abnormally activated T cells have been identified as key pathogenic cells in multiple autoimmune diseases (e.g., psoriasis, vitiligo, type 1 diabetes, multiple sclerosis, rheumatoid arthritis, Crohn's disease, ulcerative colitis, etc.) or graft-versus-host disease.

Current therapeutic agents for inflammatory or immune dysregulation diseases mainly are broad-spectrum anti-inflammatory drugs, such as glucocorticoids and nonsteroidal anti-inflammatory drugs; inflammatory factor inhibitors, such as TNFα inhibitors or IL-23-IL-17 pathway inhibitors; JAK inhibitors, such as Upadacitinib (a JAK1 inhibitor) and Ruxolitinib (a JAK1/2 inhibitor); immune cell migration inhibitors, such as natalizumab (an α4 blocker); S1PR inhibitors, such as fingolimod; and the like. However, none of these therapeutic agents can deplete pathogenic T cells at the source, and continuous medication is required to control disease progression. Therefore, a therapeutic approach that directly depletes pathogenic T cells (abnormally activated T cells) is expected to become a new breakthrough direction for the treatment of inflammatory or immune dysregulation diseases. A depleting antibody targeting CXCR6, a cell surface marker of abnormally activated T cells, holds great potential for the treatment of the aforementioned diseases.

CXCR6 belongs to the G protein-coupled receptor (GPCR) family. Proteins in this family have short and segmented extracellular domains, and antibodies targeting members of this family often exhibit a certain degree of non-specific binding to cells without corresponding antigen, resulting in poor pharmacokinetic properties. Since CXCR6 is a seven-transmembrane protein, there is an even greater need to provide an antibody that exhibits high specificity for CXCR6-expressing activated T cells. Further, given the demand for an antibody molecule with human-monkey cross-species reactivity in antibody drug development, particularly during preclinical toxicological evaluation, there remains a desire in the art to provide an anti-CXCR6 antibody with human-monkey cross-species reactivity.

### Summary of the Invention

The technical problem to be solved by the present invention is to provide an anti-CXCR6 antibody that has high affinity, high selectivity and high biological activity for CXCR6-expressing cells; exhibits no or extremely low non-specific binding activity; meanwhile, is humanized for more favorable use in clinical therapy; and is expected to possess human-monkey cross-species reactivity to facilitate its use in preclinical toxicological evaluation.

Accordingly, the present invention provides the following technical solutions.

In one aspect, the present invention provides an antibody or an antigen-binding fragment thereof, wherein the antibody or antigen-binding fragment thereof is capable of binding to CXC-family chemokine receptor 6 (CXCR6), particularly human CXCR6, with high affinity.

In the context of the present invention, unless otherwise specified, the term "CXCR6" encompasses any form of CXCR6, such as active and inactive forms, or membrane-bound and soluble forms; and encompasses any structural region of CXCR6, such as the extracellular region (ECD) and the domains comprised therein.

Particularly, the antibody or antigen-binding fragment thereof provided by the present invention comprises complementarity-determining regions (CDRs) of the heavy chain, namely heavy chain CDR1 (H-CDR1), heavy chain CDR2 (H-CDR2), and heavy chain CDR3 (H-CDR3), and complementarity-determining regions (CDRs) of the light chain, namely light chain CDR1 (L-CDR1), light chain CDR2 (L-CDR2), and light chain CDR3 (L-CDR3). According to specific embodiments of the present invention, the heavy chain CDRs comprised in the antibody or antigen-binding fragment thereof are derived from an amino acid sequence set forth in any one of SEQ ID NO: 1 to SEQ ID NO: 3; and/or the light chain CDRs comprised in the antibody or antigen-binding fragment thereof are derived from an amino acid sequence set forth in any one of SEQ ID NO: 4 to SEQ ID NO: 6.

Any amino acid sequence provided above is the amino acid sequence of the heavy chain variable region (VH) or light chain variable region (VL) of the exemplary antibody provided in the "Specific Embodiments" section of the present application. The exemplary antibody includes murine antibody, chimeric antibody, and humanized antibody. Using definition tools for complementarity-determining regions in antibody heavy-chain or light-chain known in the art (e.g., Chothia, Kabat, IMGT, Contact, CCG, etc.), those skilled in the art can readily determine the heavy-chain CDRs and the light-chain CDRs comprised therein.

Preferably, the antibody or antigen-binding fragment thereof provided by the present invention comprises heavy chain CDRs and light chain CDRs derived from the heavy chain variable region and light chain variable region shown in the following amino acid sequence pairs:
(1) SEQ ID NO:1+ SEQ ID NO:4;
(2) SEQ ID NO:2+ SEQ ID NO:5; or
(3) SEQ ID NO:3+ SEQ ID NO:6.

As described above, the CDRs in the above amino acid sequence pairs may be assigned using, for example, the CCG numbering system, as shown in Examples of the present invention.

Accordingly, in the antibody or antigen-binding fragment thereof provided by the present invention, the heavy chain CDRs and light chain CDRs are as follows:
(1) Sequentially comprising H-CDR1, H-CDR2, and H-CDR3 with the amino acid sequences shown as SEQ ID NO: 7, SEQ ID NO: 8, and SEQ ID NO: 9; and sequentially comprising L-CDR1, L-CDR2, and L-CDR3 with the amino acid sequences shown as SEQ ID NO: 11, SEQ ID NO: 12, and SEQ ID NO: 13;
(2) Sequentially comprising H-CDR1, H-CDR2, and H-CDR3 with the amino acid sequences shown as SEQ ID NO: 7, SEQ ID NO: 10, and SEQ ID NO: 9; and sequentially comprising L-CDR1, L-CDR2, and L-CDR3 with the amino acid sequences shown as SEQ ID NO: 11, SEQ ID NO: 12, and SEQ ID NO: 14; or
(3) Sequentially comprising H-CDR1, H-CDR2, and H-CDR3 with the amino acid sequences shown as SEQ ID NO: 7, SEQ ID NO: 8, and SEQ ID NO: 9; and sequentially comprising L-CDR1, L-CDR2, and L-CDR3 with the amino acid sequences shown as SEQ ID NO: 15, SEQ ID NO: 16, and SEQ ID NO: 14.

Preferably, the antibody or antigen-binding fragment thereof provided by the present invention specifically targets CXCR6, preferably human CXCR6. Optionally, the antibody or antigen-binding fragment thereof provided by the present invention may or may not possess cross-species binding activity to human, monkey (cyno), and mouse CXCR6. Preferably, the antibody or antigen-binding fragment thereof provided by the present invention has cross-species binding activity to human and monkey CXCR6.

Preferably, the antibody or antigen-binding fragment thereof provided by the present invention comprises a heavy chain variable region and a light chain variable region, both of which comprise the CDRs described above and framework regions (FRs) interspersed therebetween, with the arrangement of the regions from N-terminus to C-terminus as FR1-CDR1-FR2-CDR2-FR3-CDR3-FR4.

Further preferably, in the antibody or antigen-binding fragment thereof provided by the present invention, the heavy chain variable region may comprise the amino acid sequence set forth in any one of SEQ ID NO: 1 to SEQ ID NO: 3, or an amino acid sequence having at least 75% identity thereto; and/or the light chain variable region comprises the amino acid sequence set forth in any one of SEQ ID NO: 4 to SEQ ID NO: 6, or an amino acid sequence having at least 75% identity thereto. In the context of the present invention, differences of at most 25% in the amino acid sequence resulting from the term "at least 75% identity" may be present in any framework region of the heavy chain variable region or light chain variable region, or in any domain or sequence other than the heavy chain variable region and light chain variable region in the antibody or antigen-binding fragment thereof of the present invention. The differences may result from amino acid deletions, additions, or substitutions at any position, wherein the substitutions may be conservative substitutions or non-conservative substitutions. The term "at least 75% identity" encompasses any percentage of identity from at least 75% identity to 100% identity, such as 75%, 80%, 85%, 90%, or even 91%, 92%, 93%, 94%, 95%, 96%, 97%, 98%, 99%, or even 100% identity.

According to specific embodiments of the present invention, the antibody or antigen-binding fragment thereof provided by the present invention comprises a heavy chain variable region and a light chain variable region, wherein the heavy chain variable region and light chain variable region comprise one of the amino acid sequence combinations selected from the group consisting of:
(1) the amino acid sequence shown as SEQ ID NO: 1, or an amino acid sequence having at least 75% identity thereto; and the amino acid sequence shown as SEQ ID NO: 4, or an amino acid sequence having at least 75% identity thereto;
(2) the amino acid sequence shown as SEQ ID NO: 2, or an amino acid sequence having at least 75% identity thereto; and the amino acid sequence shown as SEQ ID NO: 5, or an amino acid sequence having at least 75% identity thereto; or
(3) the amino acid sequence shown as SEQ ID NO: 3, or an amino acid sequence having at least 75% identity thereto; and the amino acid sequence shown as SEQ ID NO: 6, or an amino acid sequence having at least 75% identity thereto.

Preferably, the antibody provided herein may be a murine antibody, a rabbit antibody, or a human antibody, and may also be a murine-derived antibody, a chimeric antibody, or a fully or partially humanized antibody. The antibody may also be referred to as a derivatized antibody, for example, an antibody obtained on the basis of an initial murine monoclonal antibody through CDR grafting, affinity maturation, site-directed mutagenesis modification, chemical modification and the like, wherein the chemical modification includes glycosylation, acetylation, polyethylene glycolization, phosphorylation, amidation, protease cleavage, conjugation to cellular ligands or effector molecules, protection and/or blocking of reactive groups, and the like. Preferably, the antigen-binding fragment of the antibody may be any form of antibody fragment, such as scFv, BsFv, dsFv, (dsFv)₂, Fab, Fab', F(ab')₂ or Fv.

In addition to the heavy-chain and/or light-chain variable regions, the antibody or antigen-binding fragment thereof provided by the present invention further comprises a heavy chain constant region (CH) and/or a light chain constant region (CL), preferably a human or murine heavy chain constant region and/or light chain constant region. Preferably, the antibody or fragment thereof comprises a heavy chain constant region of IgG, IgA, IgM, IgD, or IgE, and/or a light chain constant region of κ- or λ-type.

According to specific embodiments of the present invention, the antibody is a monoclonal antibody, preferably a murine, chimeric or humanized monoclonal antibody. According to specific embodiments of the present invention, the monoclonal antibody comprises a heavy chain constant region sequence of IgG1; and/or comprises a kappa light chain constant region.

According to specific embodiments of the present invention, the antibody of the present invention is a monoclonal antibody. Preferably, the anti-CXCR6 antibody provided by the present invention is an immunoglobulin, for example, the immunoglobulin is of human IgA, IgD, IgE, IgG, or IgM class. Further preferably, the antibody is of human IgG1 or IgG4 subclass. According to specific embodiments of the present invention, the antibody of the present invention comprises a human IgG1 heavy chain constant region and a human kappa light chain constant region, the specific sequences of which are shown in the Examples section.

Further, the anti-CXCR6 antibody or antigen-binding fragment thereof provided by the present invention exhibits antibody-dependent cell-mediated cytotoxicity (ADCC). Preferably, the anti-CXCR6 antibody or antigen-binding fragment thereof provided by the present invention has enhanced ADCC activity. For example, the anti-CXCR6 antibody is defucosylated, such as a monoclonal antibody with fucose modification removed from the N-glycosylation site. Alternatively, the anti-CXCR6 antibody provided by the present invention is a human IgG1 antibody, and possesses one or more amino acid residue mutations selected from S298A, E333A, K334A, S239D, I332E, S239D, A330L, and I332E in the Fc region of the heavy chain, wherein the amino acid residues are numbered according to the Kabat EU index. According to specific embodiments of the present invention, the anti-CXCR6 antibody provided by the present invention has a triple mutation of S298A/E333A/K334A, a double mutation of S239D/I332E, or a triple mutation of S239D/A330L/I332E.

In another aspect, the present invention provides a nucleic acid molecule comprising a nucleotide sequence encoding the antibody or antigen-binding fragment thereof of the present invention.

The expression "nucleotide sequence encoding the antibody or antigen-binding fragment thereof of the present invention" refers to a nucleotide sequence encoding the heavy chain CDRs, light chain CDRs, light chain variable region, heavy chain variable region, heavy chain and/or light chain comprised in the antibody or antigen-binding fragment thereof. For example, the nucleic acid molecule provided by the present invention comprises: a nucleotide sequence encoding each of the heavy chain CDRs and light chain CDRs comprised in the above antibody or antigen-binding fragment thereof; a nucleotide sequence encoding the heavy chain variable region and light chain variable region comprised in the above antibody or antigen-binding fragment thereof; or a nucleotide sequence encoding the heavy chain and light chain comprised in the above antibody or antigen-binding fragment thereof.

The nucleic acid molecule of the present invention may be cloned into a vector, which is then used to transform or transfect a host cell. Accordingly, in another aspect, the present invention provides a vector comprising the nucleic acid molecule of the present invention. The vector may be a eukaryotic expression vector, a prokaryotic expression vector, an artificial chromosome, a bacteriophage vector, and the like. The vector or nucleic acid molecule of the present invention may be used to transform or transfect host cells for the purposes of preservation or antibody expression. Therefore, in a further aspect, the present invention provides a host cell comprising the nucleic acid molecule and/or the vector of the present invention, or a host cell transformed or transfected with the nucleic acid molecule and/or the vector of the present invention. The host cell may be any prokaryotic or eukaryotic cell, such as a bacterial cell, an insect cell, a fungal cell or an animal cell.

The antibody or antigen-binding fragment thereof provided by the present invention may be obtained by any method known in the art. For example, the host cell provided by the present invention is cultured under conditions allowing expression of the heavy chain and light chain of the antibody. Optionally, the method further comprises a step of recovering the produced antibody.

In another aspect, the antibody or antigen-binding fragment thereof provided by the present invention may also be directly or indirectly linked to other moieties. Such other moieties are, for example, heavy chain CDRs, light chain CDRs, heavy chain variable regions, light chain variable regions, heavy chains or light chains of other antibodies. Alternatively, such other moieties are, for example, small molecule compounds, such as cytotoxic compounds for use in antibody-drug conjugates. Alternatively, such other moieties are, for example, moieties for modifying the antibody or antigen-binding fragment thereof, such as cell surface receptors, carbohydrates and polymers. Correspondingly, the present invention thus provides a conjugate, such as an antibody-drug conjugate, comprising the antibody or antigen-binding fragment thereof provided by the present invention. The present invention also provides a bispecific or multispecific antibody comprising the antibody or antigen-binding fragment thereof provided by the present invention. The present invention further provides a fusion protein comprising the antibody or antigen-binding fragment thereof provided by the present invention.

The antibody or antigen-binding fragment thereof, nucleic acid molecule, vector, host cell, conjugate, bispecific or multispecific antibody, or fusion protein provided by the present invention may be comprised in a composition, more particularly in a pharmaceutical composition, such as a pharmaceutical formulation, so as to be used for various purposes according to actual needs.

Accordingly, in another aspect, the present invention provides a composition. The composition comprises the antibody or antigen-binding fragment thereof, nucleic acid molecule, vector, host cell, conjugate, bispecific or multispecific antibody, or fusion protein provided by the present invention, and optionally a pharmaceutically acceptable excipient. The composition provided by the present invention may be formulated into various dosage forms known in the medical or pharmaceutical fields and administered in an appropriate manner.

In yet another aspect, the present invention provides use of the antibody or antigen-binding fragment thereof, nucleic acid molecule, vector, host cell, conjugate, bispecific or multispecific antibody, or fusion protein for the manufacture of a medicament for preventing, treating and/or ameliorating a disease or disorder associated with CXCR6 expression (including overexpression). In particular, the antibody or antigen-binding fragment thereof provided by the present invention can exert ADCC effect by binding to CXCR6, but is not limited thereto. For example, the antibody provided by the present invention may be a depleting antibody targeting CXCR6. Further, the disease or disorder may be acute graft-versus-host disease, chronic graft-versus-host disease, autoimmune hepatitis, Crohn's disease, ulcerative colitis, contact dermatitis, rheumatoid arthritis, vitiligo, psoriasis, psoriatic arthritis, juvenile rheumatoid arthritis, multiple sclerosis, non-alcoholic steatohepatitis, systemic lupus erythematosus, uveitis, type I diabetes, ankylosing spondylitis, myelin oligodendrocyte glycoprotein-associated disease, Graves' disease, Sjögren's syndrome, and the like.

Accordingly, the present invention further provides a method for preventing, treating and/or ameliorating a disease or disorder, comprising administering to a subject in need thereof the antibody or antigen-binding fragment thereof, nucleic acid molecule, vector, host cell, conjugate, bispecific or multispecific antibody, or fusion protein of the present invention, wherein the disease or disorder is associated with CXCR6 expression (including overexpression). Further, the disease or disorder may be acute graft-versus-host disease, chronic graft-versus-host disease, autoimmune hepatitis, Crohn's disease, ulcerative colitis, contact dermatitis, rheumatoid arthritis, vitiligo, psoriasis, psoriatic arthritis, juvenile rheumatoid arthritis, multiple sclerosis, non-alcoholic steatohepatitis, systemic lupus erythematosus, uveitis, type I diabetes, ankylosing spondylitis, myelin oligodendrocyte glycoprotein-associated disease, Graves' disease, Sjögren's syndrome, and the like. The subject may be a mammal; more preferably, the subject is a human.

The above method provided by the present invention for preventing, treating and/or ameliorating a disease or disorder depends on various factors when applied, including the specific active ingredient of the pharmaceutical composition administered, the patient's age, body weight, gender, or physical and medical condition, the severity of the condition to be treated, the route of administration, and the like.

In still another aspect, the present invention provides use of the antibody or antigen-binding fragment thereof, nucleic acid molecule, vector, host cell, conjugate, bispecific or multispecific antibody of the present invention for the manufacture of a reagent for diagnosing a disease or disorder associated with CXCR6 expression (including overexpression). Further, the disease or disorder may be acute graft-versus-host disease, chronic graft-versus-host disease, autoimmune hepatitis, Crohn's disease, ulcerative colitis, contact dermatitis, rheumatoid arthritis, vitiligo, psoriasis, psoriatic arthritis, juvenile rheumatoid arthritis, multiple sclerosis, non-alcoholic steatohepatitis, systemic lupus erythematosus, uveitis, type I diabetes, ankylosing spondylitis, myelin oligodendrocyte glycoprotein-associated disease, Graves' disease, Sjögren's syndrome, and the like.

In a further aspect, the present invention provides a method for diagnosing a disease or disorder, comprising contacting the antibody or antigen-binding fragment thereof, nucleic acid molecule, vector, host cell, conjugate, bispecific or multispecific antibody, or fusion protein of the present invention with a biological sample from a subject, wherein the disease or disorder is associated with CXCR6 expression (including overexpression). Further, the disease or disorder may be acute graft-versus-host disease, chronic graft-versus-host disease, autoimmune hepatitis, Crohn's disease, ulcerative colitis, contact dermatitis, rheumatoid arthritis, vitiligo, psoriasis, psoriatic arthritis, juvenile rheumatoid arthritis, multiple sclerosis, non-alcoholic steatohepatitis, systemic lupus erythematosus, uveitis, type I diabetes, ankylosing spondylitis, myelin oligodendrocyte glycoprotein-associated disease, Graves' disease, Sjögren's syndrome, and the like. The subject may be a mammal; more preferably, the subject is a human.

In another aspect, the present invention provides a kit, comprising the antibody or antigen-binding fragment thereof, nucleic acid molecule, vector, host cell, conjugate, or bispecific or multispecific antibody of the present invention. The kit can be used for the aforementioned prevention, treatment and/or amelioration, or the aforementioned diagnosis, or any detection of CXCR6. Depending on the intended use, the kit further comprises other reagents for prevention, treatment and/or amelioration, diagnosis or detection. For example, the kit is one used for detecting CXCR6 expression (including overexpression) in any biological sample by ELISA.

Compared with the prior art, the present invention provides a series of anti-CXCR6 murine antibodies, chimeric antibodies, as well as corresponding humanized sequences and humanized antibody molecules. Experiments have verified that the antibodies provided by the present invention can bind to CXCR6 with high affinity and high specificity by targeting different regions and sites of CXCR6, and the antibodies provided by the present invention possess cross-species binding activity to human and monkey CXCR6. Regarding the mechanism of action, without being bound by any theory, the anti-CXCR6 antibodies provided by the present invention can exert efficacy via ADCC and are depleting antibodies targeting CXCR6, thus having great potential for the treatment of inflammatory or immune dysregulation diseases and having shown significant therapeutic effects in multiple inflammatory disease models. In addition, compared with existing CXCR6 antibodies, the anti-CXCR6 antibodies provided by the present invention exhibit significantly lower non-specific cell binding, thereby possessing lower off-target toxicity and better safety profiles. Of particular importance, the present invention provides humanized anti-CXCR6 antibodies and ADCC-enhanced variants thereof, which are more favorable for clinical application.

Therefore, the anti-CXCR6 antibodies provided by the present invention have important application potential in the treatment of inflammatory or immune dysregulation diseases.

### Description of the Drawings

The embodiments of the present invention will be described in detail below with reference to the accompanying drawings, in which:
Figure 1 shows the titers of mouse serum obtained from mice immunized with human CXCR6-overexpressing cells against human CXCR6 and monkey CXCR6.
Figure 2 shows the amino acid sequence alignment of the N-terminal region and extracellular loop regions of human CXCR6 and monkey CXCR6, * indicates identical amino acids.
Figure 3 shows the titers of mouse serum against human CXCR6 and monkey CXCR6, wherein the mouse serum is obtained from a mouse immunized with cells overexpressing human CXCR6 with amino acids 2 to 13 deleted.
Figure 4 shows the binding activity of the chimeric antibody of the present invention to different CXCR6-overexpressing cells or blank cells, wherein 4A: HEK293-huCXCR6 cells; 4B: CHOS-huCXCR6-1T48 cells; 4C: HEK293-cynoCXCR6 cells; 4D: CHOS-mCXCR6 cells.
Figure 5 shows the ADCC activity of the chimeric antibody of the present invention against different CXCR6-overexpressing cells or blank cells, wherein 5A: HEK293-huCXCR6 cells; 5B: CHOS-huCXCR6-1T48 cells; 5C: HEK293-cynoCXCR6 cells.
Figure 6 shows the binding activity of the humanized antibody hz22C6-H3L3 of the present invention to different CXCR6-overexpressing cells or blank cells, wherein 6A: CHOS-huCXCR6-1T48 cells; 6B: HEK293-cynoCXCR6 cells.
Figure 7 shows the non-specific binding activity of the humanized antibody hz22C6-H3L3 of the present invention to different blank cells and ssDNA, wherein 7A: CHOS-Blank cells; 7B: HEK293-Blank cells; 7C: ssDNA.
Figure 8 shows the binding activity of the humanized antibody hz22C6-H23L30 of the present invention to different cells or ssDNA, wherein 8A: HEK293-huCXCR6 cells; 8B: HEK293-cynoCXCR6 cells; 8C: HEK293-Blank cells; 8D: ssDNA.
Figure 9 shows the ADCC activity of the humanized antibody hz22C6-H23L30 of the present invention against different CXCR6-overexpressing cells or blank cells, wherein 9A: HEK293-huCXCR6 cells; 9B: HEK293-cynoCXCR6 cells; 9C: HEK293-Blank cells.
Figure 10 shows the activities of different ADCC-enhancing versions of the humanized antibody hz22C6-H23L30 of the present invention, wherein 10A: binding activity to CHOS-huCXCR6-1T48 cells; 10B: ADCC activity against CHOS-huCXCR6-1T48 cells.
Figure 11 shows the epitope analysis results of clone 22C6 of the present invention, wherein 11A: Relative binding intensity of the humanized antibody hz22C6-H3L3 to huCXCR6 and its human-mouse chimeric versions with different regions; 11B: Relative binding intensity of the humanized antibody hz22C6-H3L3 to huCXCR6 and its different point mutant versions in the ECL2 and ECL3 thereof.
Figure 12 shows the plasma concentration profiles of humanized antibodies hz22C6-H3L3 and hz22C6-H23L30 of the present invention in mice.
Figure 13 shows the plasma concentration profiles of humanized antibody hz22C6-H23L30 of the present invention in cynomolgus monkeys.
Figures 14 to 16 show the pharmaceutical efficacy of humanized antibodies hz22C6-H3L3 and hz22C6-H23L30 of the present invention in a mouse EAE model.
Figure 17 shows the pharmaceutical efficacy of the humanized antibody hz22C6-H23L30 of the present invention in a mouse CIA model, wherein 17A: CIA clinical score; 17B: body weight profile of mice; 17C: CIA pathological score; 17D: typical HE-stained pathological image of CIA.
Figure 18 shows the pharmaceutical efficacy of the humanized antibody hz22C6-H23L30 of the present invention in a mouse GvHD model.
Figure 19 shows the pharmaceutical efficacy of the humanized antibody hz22C6-H23L30 (Fuc⁻) of the present invention in a mouse IBD model, wherein 19A: clinical score of IBD disease activity; 19B: statistical graph of the large intestine weight-to-length ratio; 19C: photograph of the large intestine taken at the experimental endpoint; 19D: typical HE-stained pathological image of the large intestine; 19E: flow cytometry results.
Figure 20 shows the pharmaceutical efficacy of the humanized antibody hz22C6-H23L30 of the present invention in a mouse vitiligo model, wherein 20A: vitiligo score; 20B: typical photographs of mice.
Figure 21 shows the pharmaceutical efficacy of anti-muCXCR6 antibody in a mouse T1D model, wherein 21A: T1D incidence rate; 21B: blood glucose concentration profile.

### Specific Embodiments

The present invention is illustrated below with reference to specific Examples. Those skilled in the art will understand that these Examples are merely illustrative of the present invention and do not in any way limit the scope of the invention.

Unless otherwise specified, all experimental methods in the following Examples are conventional methods. Unless otherwise specified, the raw materials, reagents, and materials used in the following Examples are commercially available products.

In the Examples:
Human CXCR6 (huCXCR6 or hCXCR6): see UniProtKB-O00574;
Monkey CXCR6 (cynoCXCR6): see UniProtKB-Q9BDS6;
Murine CXCR6 (muCXCR6 or mCXCR6): see UniProtKB-Q9EQ16.

Control antibody Beacon2-G5: derived from patent publication WO2022251853A1, with the variable region sequences as set forth below, and obtained by constructing with a human IgG1 heavy chain constant region and a human kappa light chain constant region.
Heavy chain variable region [SEQ ID NO: 17]: 
EVQLVESGGDLVKPGGSLKLSCAASGFTFSSYGMSWVRQTPDKRLEWVATISSRG
SYTYYPDSVKGRLTISRENAKNTLSLQMSSLKSEDTAMYYCARRVFPYWFFDVWGAG
TTVTVSS
Light chain variable region [SEQ ID NO: 18]: 
DIVMTQSHKFMSTSVGDRVSITCKASQDVSTAVAWYQQKSGQSPKLLIYSASYRY
TGVPDRFTGSGSGTDFTFTISSVQAEDLAVYYCQQHYTTPYTFGGGTKLEIK
Human IgG1 heavy chain constant region [SEQ ID NO:19]: 
ASTKGPSVFPLAPSSKSTSGGTAALGCLVKDYFPEPVTVSWNSGALTSGVHTFPAV
LQSSGLYSLSSVVTVPSSSLGTQTYICNVNIIKPSNTKVDKRVEPKSCDKTIITCPPCPAPE
LLGGPSVFLFPPKPKDTLMISRTPEVTCVVVDVSHEDPEVKFNWYVDGVEVHNAKTKP
 
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK
Human kappa light chain constant region [SEQ ID NO:20]:

### Example 1: Mouse antibody screening

### 1.1 Immunization of mice with human CXCR6

Using CHO-S cells, a cell strain overexpressing human CXCR6, abbreviated as "CHOS-huCXCR6-1T19" cell, was established (established by Beijing Kohnoor Science & Technology Co., Ltd., Clone No. 1T19). The cells were used to immunize Balb/c mice (female, 6-8 weeks old, purchased from Shanghai BK/KY Biotechnology Co., Ltd.). After three immunizations (administered at 2-week intervals, intraperitoneal injection of 100 µL PBS suspension containing 1E7 cells each time of immunization), orbital blood was collected from the mice.

Using HEK293 cells, cell lines expressing human CXCR6 and monkey CXCR6 were established, abbreviated as "HEK293-huCXCR6" and "HEK293-cynoCXCR6" cells, respectively. Serum titration tests were performed using HEK293-huCXCR6, HEK293-cynoCXCR6, and HEK293-Blank cells. Specifically, PBS suspensions of the three cell types were prepared at a density of 4E6/mL and seeded into a 96-well plate at 50 ul/well. Mouse serum was diluted at a dilution ratio of 1:50 with PBS and added to the 96-well plate at 50 ul/well. The 96-well plate was incubated at 4° C for 1 hour. Subsequently, the supernatant was removed by centrifugation, and the wells were washed twice with PBS. A 1:1000 dilution of secondary antibody (APC-goat anti-mouse IgG (H+L), Jackson Immuno, 115-136-146) was added to the 96-well plate, incubated for 30 minutes, then centrifuged and discarded the supernatant. The wells were washed twice with PBS, then 30 ul of PBS was added to each well and mixed thoroughly. The serum titer was detected using an IQue Screener PLUS (Sartorius) flow cytometer.

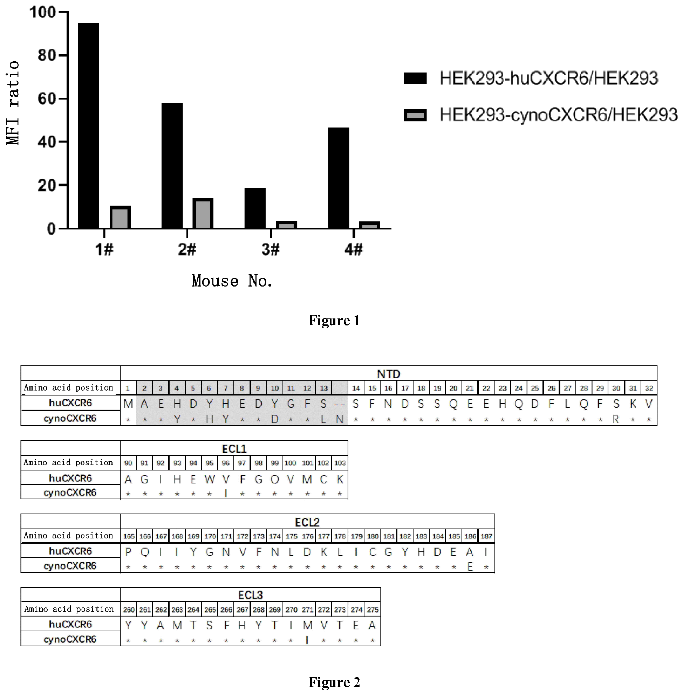

The results were shown in Figure 1 (data were presented as "FACS signal value of the overexpression cell line / FACS signal value of the blank cell line" to demonstrate the target-specific titration response). The results indicated that the sera from four mice exhibited strong specific titration responses to huCXCR6, but relatively weak specific titration responses to cynoCXCR6.

### 1.2 Immunization of mice using human CXCR6 with amino acids at positions 2-13 deleted

Preclinical toxicological evaluation is a critical component of drug development, and molecules with cross-reactivity across human and cynomolgus species can greatly facilitate such toxicological evaluation. Given that the sera from mice immunized with CHOS-huCXCR6-1T19 exhibited low titers against cynoCXCR6, the immunization cell line was redesigned to develop antibodies with strong binding capacity to both human and monkey CXCR6.

Conservation analysis of the extracellular domains of huCXCR6 and cynoCXCR6 revealed that the N-terminal domain (NTD) of huCXCR6 and cynoCXCR6 exhibited significant differences, whereas the extracellular loops (ECL) 1-3 showed high conservation. The alignment results were shown in Figure 2.

NIH/3T3 cells were used to construct a cell line overexpressing human CXCR6 with amino acids at positions 2-13 (corresponding to the gray-shaded residues in Figure 2) deleted. The cell line was designated as "NIH/3T3-huCXCR6 (δ2-13AA)" cell. NIH/3T3 cells were employed to further minimize immune responses against other irrelevant cellular antigens. Following the same experimental procedures described above, Balb/c mice were immunized with NIH/3T3-huCXCR6 (δ2-13AA) cells, and serum titers were measured.

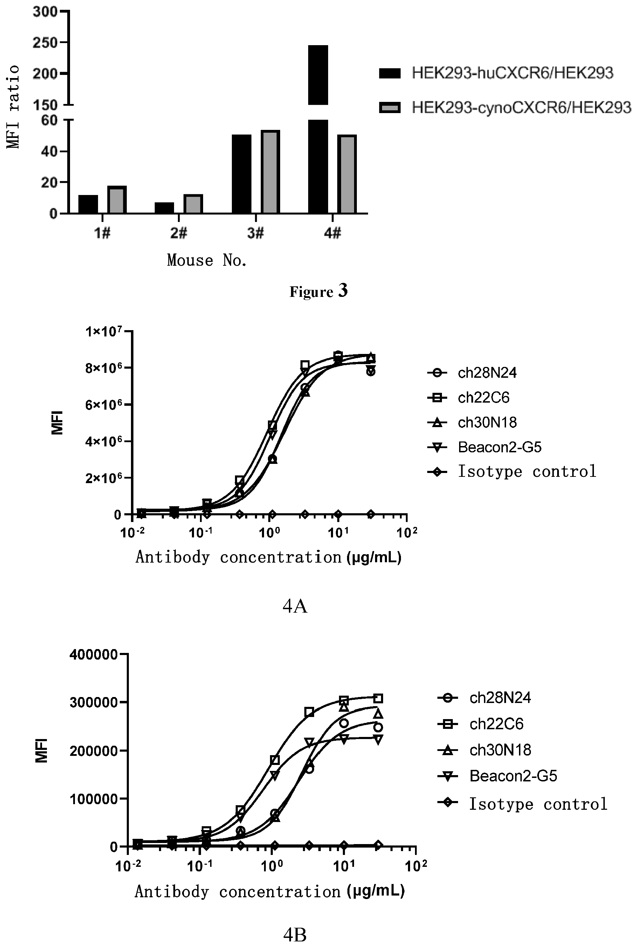

The results were shown in Figure 3, demonstrating that sera from mice immunized with NIH/3T3-huCXCR6(δ2-13AA) cells exhibited strong specific titer responses against both huCXCR6 and cynoCXCR6.

### 1.3 Screening of hybridoma clones

On the third day after the mice received a booster immunization with the corresponding overexpression cell lines, the mice were sacrificed. The spleens and lymph nodes were harvested and ground, and the resulting cells were pretreated and electrofused with mouse myeloma cells. After 10 days of culture in HAT medium, hybridoma supernatants were screened via flow cytometry using HEK293-huCXCR6, HEK293-cynoCXCR6 and HEK293-Blank cell lines. The experimental procedure was the same as that described above.

More than 100 hybridoma clones exhibiting cross-binding activity against huCXCR6 and cynoCXCR6 were finally obtained.

### Example 2: Preparation and activity characterization of human-mouse chimeric antibodies

### 2.1 Vector construction and antibody production

Antibody gene sequences of hybridoma clones were amplified using degenerate primers targeting mouse light and heavy chains. The resulting light- and heavy-chain amplification fragments were directly ligated into the pTT5 expression vector comprising the human kappa light-chain constant region [SEQ ID NO:20] and human IgG1 heavy-chain constant region [SEQ ID NO:19]. The ligation products were transformed into competent DH5α cells (Yestern, Catalog No. E607-80VL), followed by plating. Single colonies were picked and sequenced.

After analyzing and obtaining correct antibody light- and heavy-chain gene sequences, paired transient transfection and expression were performed using suspension-adapted HEK293 cells. The heavy and light chain plasmids at a ratio of 2:3 were transfected into HEK293 cells at a density of 2E6/ml using PEI reagent (Polysciences, Catalog No. 24885) (1 µg plasmid:4 µg PEI). The transfected cells were cultured at 37°C, 5% CO₂ for 5-7 days. The supernatants were collected and purified using a purification column packed with rProtein A Sepharose (GE Healthcare Biosciences, Catalog No. 127903). The purified samples were sterile-filtered through a 0.22 µm filter, and the concentrations of the purified antibodies were determined using a Nanodrop One (Thermo Fisher Scientific).

### 2.2 Analysis of binding activity of chimeric antibodies

A series of human-mouse chimeric antibodies were preliminarily screened and identified. Three antibody molecules with better activity were selected and designated as "ch28N24", "ch22C6", and "ch30N18" respectively, and the corresponding clones were named as "28N24", "22C6", and "30N18" respectively.

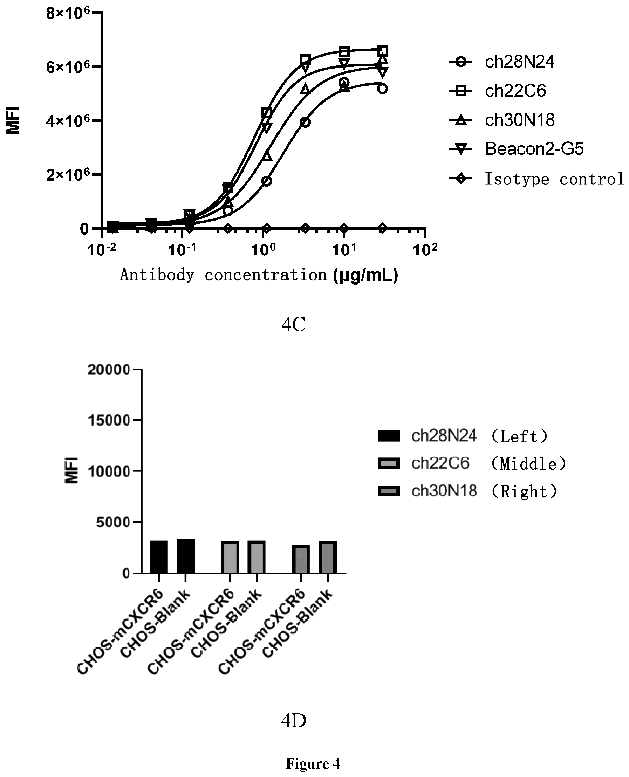

The binding activity of these antibody molecules was characterized via FACS using several cell lines overexpressing huCXCR6, cynoCXCR6, and muCXCR6, with a blank cell line included for non-specific binding assessment. Beacon2-G5 was used as the control antibody in the experiments. Specifically, a PBS suspension of each cell line was prepared at a density of 4E6/mL and seeded into a 96-well plate at 50 µL per well. Each antibody was serially diluted 2-fold in PBS to designated concentrations, and added to the 96-well plate at 50 µL/well. The plate was incubated at 4°C for 1 hour. Subsequently, the supernatant was removed by centrifugation, and the wells were washed twice with PBS. 1:1000 dilution of a secondary antibody (APC-goat anti-human IgG Fcγ, JacksonImmuno, 109-135-098) was added to the 96-well plate, and incubated for 30 minutes. Supernatants were discarded after centrifugation, and cells were washed twice with PBS. Next, 30 µL of PBS was added to each well and mixed thoroughly, then IQue Screener PLUS (Sartorius) flow cytometer was used to detection. FACS binding curves were shown in Figure 4, and the binding EC50 values were summarized in Table 1.

The results indicated that all three antibody molecules exhibited excellent binding activity to huCXCR6 and cynoCXCR6, with EC50 values ranging from 0.7 to 2.6 µg/mL (Figures 4A-C; Table 1). Meanwhile, none of the three antibody molecules bound to muCXCR6 (Figure 4D). Therefore, the experiment verified that the chimeric antibodies provided by the present invention all possessed good human-monkey cross-binding activity.

**Table 1. Binding activity of chimeric antibodies to cells expressing target proteins**

| Antibody name | FACS binding activity, EC50 (µg/mL) | | |
|---|---|---|---|
| | HEK293-huCXCR6 cells | HEK293-cynoCXCR6 cells | CHOS-huCXCR6-1T48 cells |
| ch28N24 | 1.48 | 1.79 | 2.38 |
| ch22C6 | 0.93 | 0.79 | 0.89 |
| ch30N18 | 1.65 | 1.25 | 2.61 |
| Beacon2-G5 | 1.03 | 0.82 | 0.76 |

### 2.3 Analysis of ADCC activity of chimeric antibodies

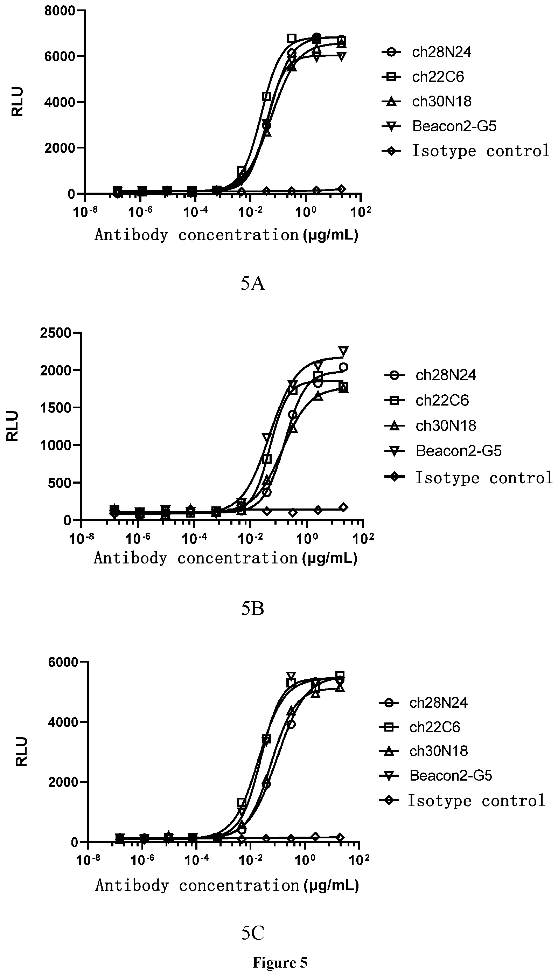

Antibody-dependent cell-mediated cytotoxicity (ADCC) is the primary mechanism of action for anti-CXCR6 depleting antibodies in the treatment of inflammatory diseases. Accordingly, ADCC activity analysis of the chimeric antibody molecules provided by the present invention was performed using ADCC reporter cells. Specifically, CXCR6-overexpressing cell lines or a control cell line were used as target cells and seeded at 2E4 cells per well in a 96-well plate. The initial working concentration of the test antibody was 20 µg/mL, serially diluted 8-fold to create a total of 10 concentration gradients. The diluted antibodies were added to the cells and mixed thoroughly, followed by incubation at 37°C in a 5% CO₂ incubator for 1 hour. Effector cells Jurkat-CD16A/NF (purchased from Genomeditech (Shanghai) Co., Ltd.) were added at an effector-to-target ratio of 7.5:1, with 1.5E5 effector cells per well, and gently mixed to ensure sufficient contact between effector and target cells, then incubated at 37°C in a 5% CO₂ incubator for 5 hours. Bio-Lite^{™} detection reagent (Vazyme, Catalog No. DD1201) was added to measure the fluorescence signal from the wells of the plate. ADCC activities were shown in Figure 5.

The results indicated that all three antibody molecules exhibited potent ADCC activity against both huCXCR6- and cynoCXCR6-overexpressing cell lines.

### Example 3: Humanization engineering on the mouse antibody derived from clone 22C6

### 3.1 Humanization

The mouse antibody 22C6 derived from clone 22C6 was humanized via CDR grafting, with CDRs defined according to the CCG numbering system. The selected humanization templates were shown in Table 2; and the humanized light- and heavy-chain sequences of different versions were shown in Table 3.

**Table 2. Light- and heavy-chain humanization templates used for the humanization of the mouse antibody 22C6**

| Parental heavy chain | V-region template | J-region template | Parental light chain | V-region template | J-region template |
|---|---|---|---|---|---|
| 22C6_VH | IGHV3-23*01 | IGHJ6-1 | 22C6_VK | IGKV1-12*01 | IGKJ4-1 |

**Table 3. Sequences of mouse antibody 22C6 and its various humanized versions (CDRs were shown in bold and underlined)**

| Sequence name | Sequence |
|---|---|
| >22C6_VH [VH of mouse antibody 22C6; SEQ ID NO:1] | EVKLEESGGDLVKPGGSLKLSCAASGFTFSSYGMSWVRQTPDKRL EWVATISSGGSYTYYPDSVKGRFIISRDNAKNTLYLQVSSLKSEDT AMYYCSRLYGNYPFFTMDYWGQGTSVTVSS |
| | H-CDR1:GFTFSSYGMS[SEQ ID NO:7] |
| | H-CDR2:TISSGGSYTYYPDSVKG[SEQ ID NO:8] |
| | H-CDR3:LYGNYPFFTMDY[SEQ ID NO:9] |
| >22C6_VH_hz3 [humanized sequence; SEQ ID NO:2] | EVQLLESGGGLVQPGGSLRLSCAASGFTFSSYGMSWVRQAPGKGL EWVATISSGGSYTYYPDTVKGRFTISRDNSKNTLYLQMNSLRAED TAVYYCSRLYGNYPFFTMDYWGQGTTVTVSS |
| | H-CDR1:GFTFSSYGMS[SEQ ID NO:7] |
| | H-CDR2:TISSGGSYTYYPDTVKG[SEQ ID NO:10] |
| | H-CDR3:LYGNYPFFTMDY[SEQ ID NO:9] |
| >22C6_VH_hz23 [humanized sequence; SEQ ID NO:3] | EVQLLESGGGLVQPGGSLRLSCAASGFTFSSYGMSWVRQAPGKGL EWVSTISSGGSYTYYPDSVKGRFTISRDNSKNTLYLQMNSLRAEDT AVYYCSRLYGNYPFFTMDYWGQGTTVTVSS |
| | H-CDR1:GFTFSSYGMS[SEQ ID NO:7] |
| | H-CDR2:TISSGGSYTYYPDSVKG[SEQ ID NO:8] |
| | H-CDR3:LYGNYPFFTMDY[SEQ ID NO:9] |
| >22C6_VK [VL of mouse antibody 22C6; SEQ ID NO:4] | DVVMTQSQKFMSTSVGDRVSVTCKASQNVRTNVAWYQQKPGQS PKALIHSASYRNTGVPDRFTGSGSGTDFTLTINNVQSEDLAEYFCQ QYNSYPYTFGGGTKLEIK |
| | L-CDR1:KASQNVRTNVA[SEQ ID NO:11] |
| | L-CDR2:SASYRNT[SEQ ID NO:12] |
| | L-CDR3:QQYNSYPYT[SEQ ID NO:13] |
| >22C6_VK_hz3 [humanized sequence; SEQ ID NO:5] | DIQMTQSPSSVSASVGDRVTITCKASQNVRTNVAWYQQKPGKAPK LLIYSASYRNTGVPSRFSGSGSGTDFTLTISSLQPEDFATYYCQQYN TYPYTFGGGTKVEIK |
| | L-CDR1:KASQNVRTNVA[SEQ ID NO:11] |
| | L-CDR2:SASYRNT[SEQ ID NO:12] |
| | L-CDR3:QQYNTYPYT[SEQ ID NO:14] |
| >22C6_VK_hz30 [humanized sequence; SEQ ID NO:6] | DIQMTQSPSSVSASVGDRVTITCKASQNVRSWLAWYQQKPGKAPK LLIYSASSRQSGVPSRFSGSGSGTDFTLTISSLQPEDFATYYCQQYNT YPYTFGGGTKVEIK |
| | L-CDR1:KASQNVRSWLA[SEQ ID NO:15] |
| | L-CDR2:SASSRQS[SEQ ID NO:16] |
| | L-CDR3: QQYNTYPYT [SEQ ID NO:14] |

### 3.2 Binding activity analysis of the humanized antibody hz22C6-H3L3 (VH: 22C6_VH_hz3; VL: 22C6_VK_hz3)

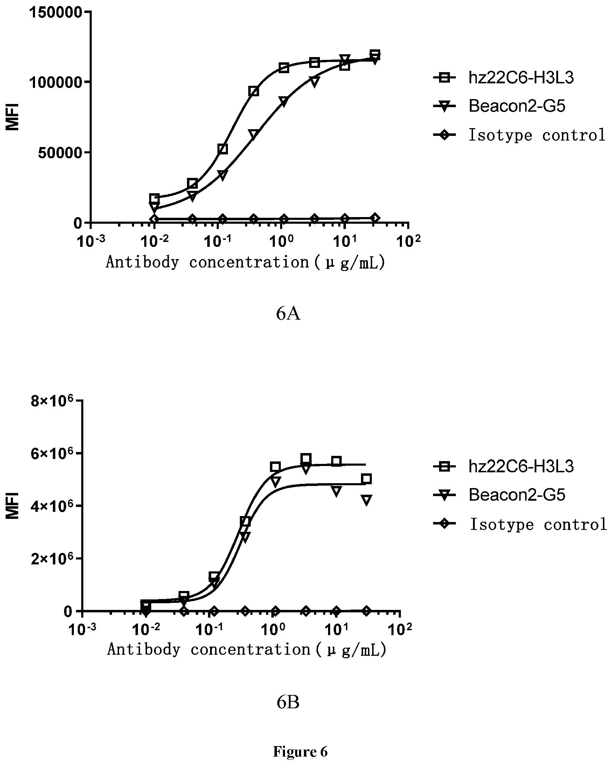

The humanized antibody molecule of hz22C6-H3L3 was generated as described in Example 2, and its binding activity was assayed by FACS. The FACS binding curves were shown in Figure 6, demonstrating that hz22C6-H3L3 exhibited very strong binding activity to huCXCR6 and cynoCXCR6.

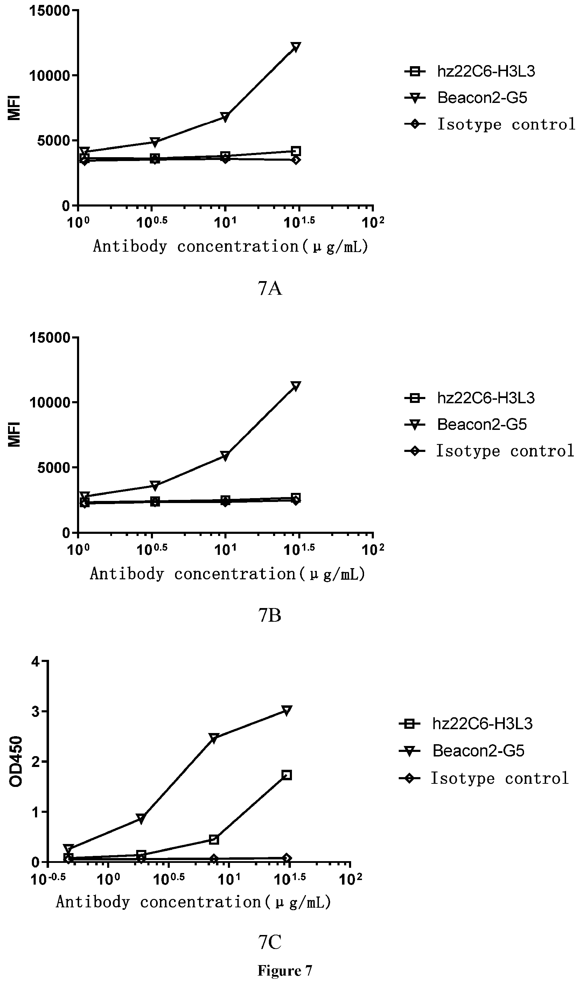

Since CXCR6 belongs to the GPCR family of seven-transmembrane proteins, the extracellular segments of proteins in this family are generally short and fragmented. Antibodies targeting this family often exhibit a certain degree of non-specific binding to blank cells and biomacromolecules (proteins, lipids, DNA, etc.). The binding activity of hz22C6-H3L3 was tested using blank cell lines without CXCR6 expression (HEK293-Blank and CHOS-Blank). The results showed that hz22C6-H3L3 exhibited no non-specific binding to blank cells, whereas the control molecule Beacon2-G5 at high concentrations exhibited strong non-specific binding (Figures 7A and 7B). Meanwhile, the non-specific binding activity of hz22C6-H3L3 to ssDNA was assessed using an ELISA assay. Specifically, ssDNA (Sigma, Catalog. No. D8899) was used as the coating antigen, and anti-human IgG, Fcγ fragment specific (Jackson Immuno, 109-135-098) was used as the secondary antibody. The results showed that hz22C6-H3L3 at the highest concentration of 30 µg/mL exhibited a low degree of non-specific binding, while the control molecule Beacon2-G5 displayed very significant non-specific binding at a concentration of 7.5µg/mL (Figure 7C).

### 3.3 Binding activity analysis of the humanized antibody hz22C6-H23L30 (VH: 22C6_VH_hz23; VL: 22C6_VK_hz30)

Further, to increase the humanization degree of hz22C6-H3L3 and simultaneously eliminate its binding to ssDNA, further mutations were designed on the light and heavy chains of hz22C6-H3L3, resulting in the molecule hz22C6-H23L30. The humanization degrees of both the light and heavy chains of hz22C6-H23L30 were improved (Table 4).

**Table 4. Humanization degrees of humanized antibodies**

| Light-chain name | Humanization degree (%) | Heavy-chain name | Humanization degree (%) |
|---|---|---|---|
| >IGKV1-12*01+>IGKJ4-1 | -- | >IGHV3-23*01+>IGHJ6-1 | -- |
| >22C6_VK_hz3 | 85 | >22C6_VH_hz3 | 89 |
| >22C6_VK_hz30 | 91 | >22C6_VH_hz23 | 91 |

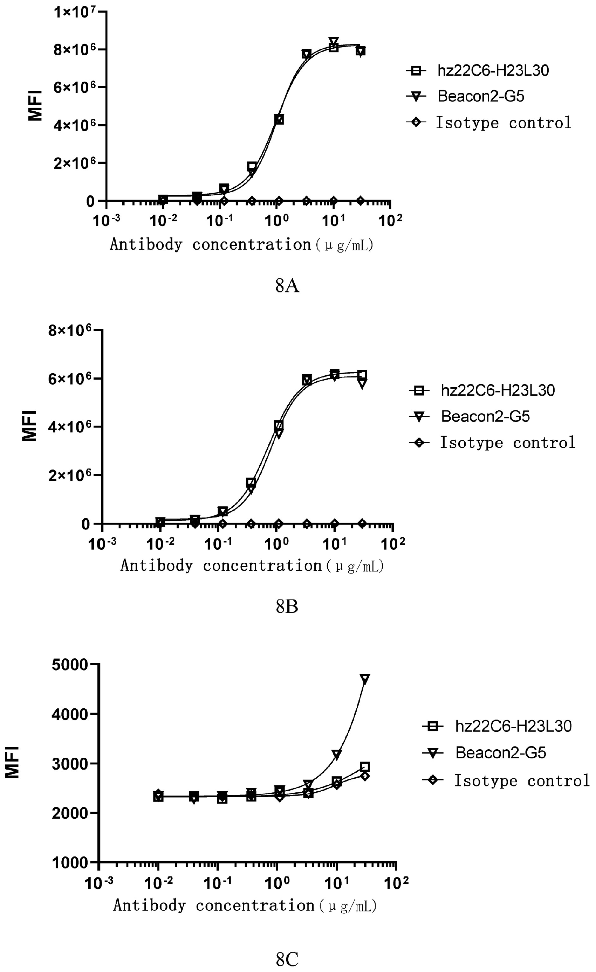

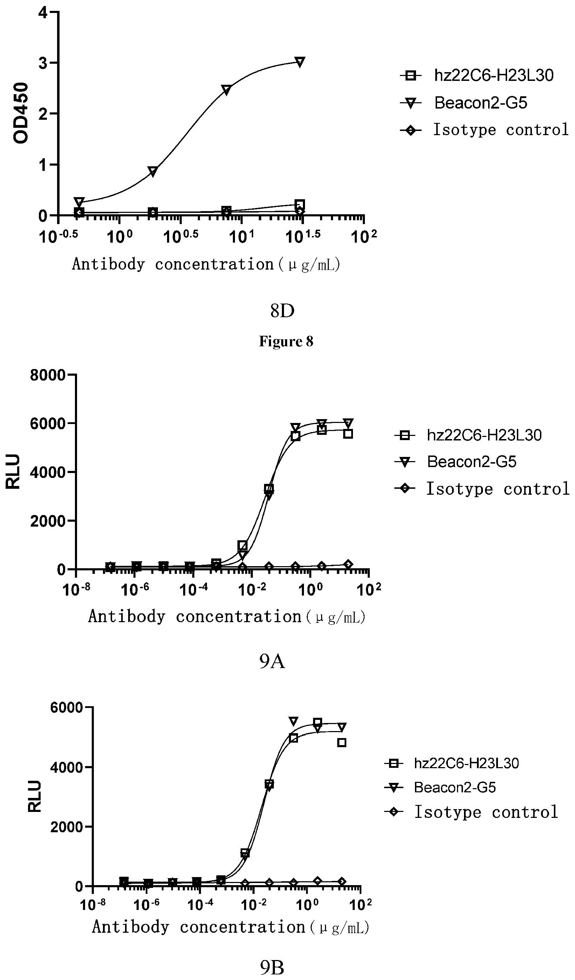

Binding activity analysis via FACS was performed on hz22C6-H23L30. The results showed that hz22C6-H23L30 exhibited very strong binding activity to huCXCR6 and cynoCXCR6 (Figures 8A and 8B), while displayed no non-specific binding to blank cells HEK293-blank (Figure 8C). An ELISA was performed to test the non-specific binding of hz22C6-H23L30 to ssDNA. The results showed that hz22C6-H23L30 exhibited no non-specific binding to ssDNA even at a high concentration (30 µg/mL).

### 3.4 ADCC activity analysis of hz22C6-H23L30

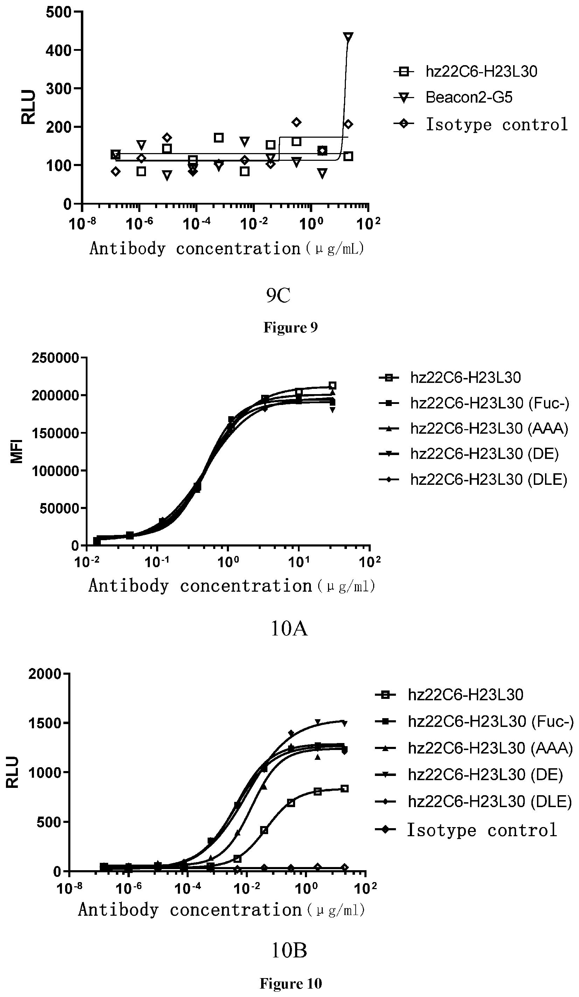

ADCC activity of hz22C6-H23L30 was analyzed as described in Example 2. The ADCC activities were shown in Figures 9A and 9B. The results showed that hz22C6-H23L30 exhibited very strong ADCC activity against both huCXCR6- and cynoCXCR6-overexpressing cell lines, and displayed no ADCC activity against blank cells (Figure 9C), whereas the control molecule Beacon2-G5 produced an ADCC signal at high concentrations, suggesting a potential off-target cytotoxicity in vivo.

### Example 4: Activity assay of molecules with enhanced ADCC activity

According to published information, the ADCC activity of antibodies is influenced by Fc receptor glycoforms and amino acid mutations at specific Fc sites. To obtain antibody molecules with enhanced ADCC activity, the inventors designed and prepared antibody molecules based on hz22C6-H23L30 to enhance ADCC activity. A total of four different version molecules with enhanced ADCC activity were obtained. Among them, hz22C6-H23L30 (Fuc⁻) was a molecule lacking fucose in the N- glycosylation modification of the Fc region; hz22C6-H23L30 (AAA) was a molecule with triple mutations (S298A/E333A/K334A) introduced into the heavy chain; hz22C6-H23L30(DE) was a molecule with S239D/I332E double mutations introduced into the heavy chain, and hz22C6-H23L30(DLE) was a molecule with S239D/A330L/I332E triple mutations introduced into the heavy chain (wherein amino acid residues were numbered according to the Kabat EU index).

Results from FACS and ADCC activity assays indicated that the binding activity of the four version molecules with enhanced ADCC activity to huCXCR6 was consistent with that of hz22C6-H23L30 (Figure 10A), while their ADCC activity was significantly enhanced compared to hz22C6-H23L30 (Figure 10B), suggesting a superior capacity to deplete CXCR6-positive cells in vivo.

### Example 5: Epitope analysis of clone 22C6

Epitope analysis of clone 22C6 was performed using hz22C6-H3L3.

### 5.1 Binding region analysis

Given that clone 22C6 does not bind to muCXCR6, the N-terminus and extracellular loops 1-3 of muCXCR6 were individually substituted into huCXCR6 to construct pCDNA3.1 transient expression vectors for human-mouse chimeric CXCR6 genes. A 6*his tag was added to the N-terminus of huCXCR6 gene and each chimeric gene for normalization of subsequent experimental data. All vectors were transiently expressed in HEK293 cells. Forty-eight hours after transfection, flow cytometric analysis was performed using an anti-His-tag antibody (Biolegend, Catalog No. 362605) and hz22C6-H3L3, following the same method described above.

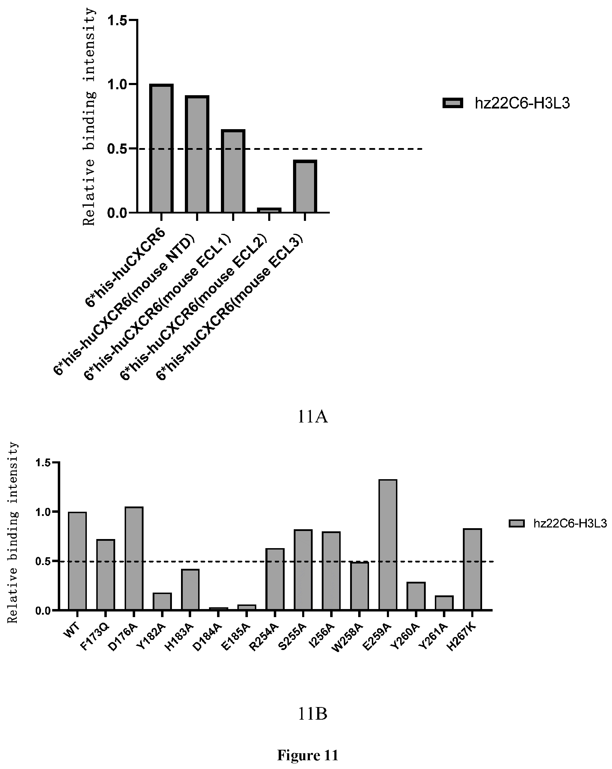

By normalizing the His-tag signal values relative to the huCXCR6 signal value, the normalized results were shown in Figure 11A. The results indicated that the binding epitope of clone 22C6 was primarily located on ECL2, with minor contributions from ECL1 and ECL3.

### 5.2 Binding site analysis

Further mutation analysis was performed on human-mouse divergent amino acids in ECL2 and ECL3 to identify the key epitope of clone 22C6. A total of 14 His-tagged point mutation transient expression vectors were constructed, including F173Q, D176A, Y182A, H183A, D184A, E185A, R254A, S255A, I256A, W258A, E259A, Y260A, Y261A, H267K, and performed transient expression in HEK293 cells followed by flow cytometry analysis. The results were shown in Figure 11B, indicating that the key epitope residues of clone 22C6 were Y182, H183, D184, E185, Y260, and Y261.

### Example 6: Pharmacokinetic analysis of humanized antibodies

### 6.1 Pharmacokinetic analysis in mice

Pharmacokinetic (PK) analysis was performed in mice for different humanized antibody molecules derived from clone 22C6.

Female Balb/c mice older than six weeks of age (purchased from Shanghai BK/KY Biotechnology Co., Ltd.) were used. The dosage was a single intravenous injection of 200 µg per mouse, with four mice per group. Blood samples of approximately 50 µL were then collected at 1 h, 4 h, 8 h, 24 h, 48 h, 96 h, 120 h, 144 h, 192 h, 240 h, 288 h and 336 h after dosing; serum was separated and stored at -80°C.

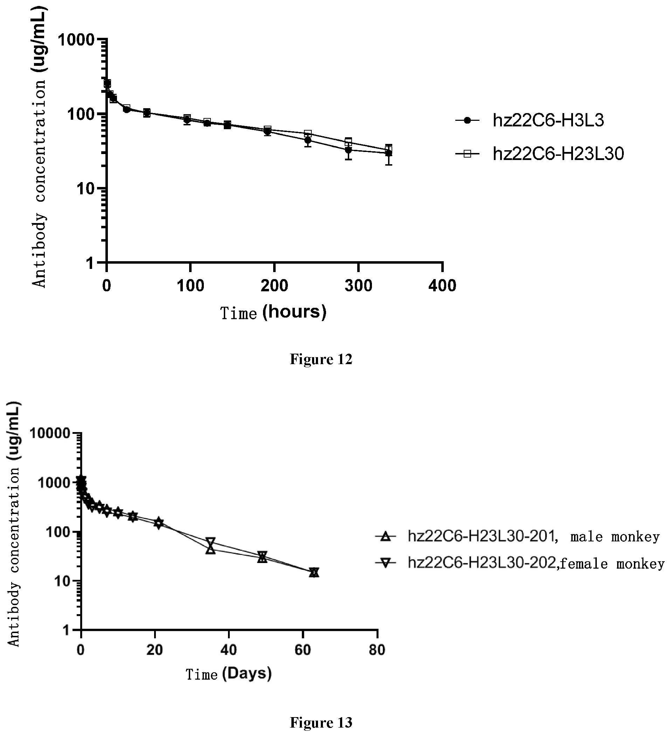

Serum concentrations of each antibody molecule at different time points were detected by ELISA. Anti-human IgG Fab monoclonal antibody (Sigma, Catalog No. I5260-1ML) was used as the coating agent, and anti-human IgG, Fcγ fragment specific (Jackson Immuno, Catalog No. 109-135-098) was used as the secondary antibody. The serum concentration profiles of the respective antibodies were shown in Figure 12, and PK analysis results were presented in Table 5. Specifically, hz22C6-H23L30 exhibited a half-life (t₁/₂) of 178.06 h, demonstrating a favorable PK metabolic property in mice.

**Table 5. Pharmacokinetic analysis results of humanized antibodies in mice**

| PK parameter (Unit) | hz22C6-H3L3 | hz22C6-H23L30 |
|---|---|---|
| t_{1/2} (hours) | 153.37 | 178.06 |
| Cmax (µg/ml) | 256.48 | 255.53 |
| AUC-192 (µg/ml*hour) | 23435.82 | 24936.78 |

### 6.2 Pharmacokinetic analysis in cynomolgus monkeys

PK analysis of hz22C6-H23L30 was performed in cynomolgus monkeys.

Two cynomolgus monkeys, one female and one male, were used. A dose of 20 mg/kg was administered via intravenous infusion within 30 minutes. Blood samples of approximately 500 µL were collected immediately after the infusion, as well as at 1 h, 2 h, 4 h, 8 h, 24 h, 48 h, 72 h, Day 5, Day 7, Day 10, Day 14, Day 21, Day 35, Day 49, and Day 63 after the infusion; serum was separated and stored at -80°C.

Serum concentrations of each antibody molecule at respective time points were detected by ELISA. Goat anti-human IgG(H+L), minX Monkey (BERHYL, Catalog No. A80-319A) was used as the coating agent, and goat anti-human IgG, Monkey ads-HRP (SouthernBiotech, 2049-05) was used as the secondary antibody. Finally, the resulting serum concentration profile of the antibody was shown in Figure 13, and PK analysis results were presented in Table 6.

**Table 6. Pharmacokinetic analysis results of the humanized antibodies in cynomolgus monkeys**

| PK parameter (Unit) | hz22C6-H23L30 (Male monkey) | hz22C6-H23L30 (Female monkey) |
|---|---|---|
| t_{1/2} (days) | 12.37 | 13.65 |
| Cmax (µg/ml) | 1212.00 | 1082.00 |
| AUC-D63 (µg/ml*day) | 8521.56 | 7725.94 |

The t_{1/2} of hz22C6-H23L30 was approximately 13 days, indicating a good PK metabolic property in cynomolgus monkeys.

### Example 7: Pharmaceutical efficacy of the humanized antibodies

### 7.1 Pharmaceutical efficacy in experimental autoimmune encephalomyelitis model (EAE model)

Since the screened antibody molecules did not bind to muCXCR6, huCXCR6-knock inC57BL/6J mice (Shanghai Model Organisms Center, Inc.) were used for modeling. The modeling procedure was briefly described as follows: mice were anesthetized by inhalation of a mixture of 1-4% isoflurane and oxygen (at a flow rate of 1-2 L/min), followed by subcutaneous injection into the back with 100 µL emulsion (emulsion composition: 2 mg/mL MOG₃₅₋₅₅ and complete Freund's adjuvant containing 4 mg/mL *Mycobacterium tuberculosis,* mixed by high-speed homogenization at an equal volume) to immune. Meanwhile, mice were intraperitoneally injected with pertussis toxin (1 µg/mL, 200 µL), and an equal dose of pertussis toxin was intraperitoneally re-administered 48 hours later.

Following modeling, mice were weighed daily and clinically scored for EAE. The scoring criteria were as follows:
Score 0: Normal, with no obvious disease manifestations;
Score 1: Flaccid tail, or hind-limb weakness;
Score 2: Flaccid tail accompanied by hind-limb weakness;
Score 3: Partial hind-limb paralysis;
Score 4: Complete hind-limb paralysis;
Score 5: Moribund state or death due to EAE.

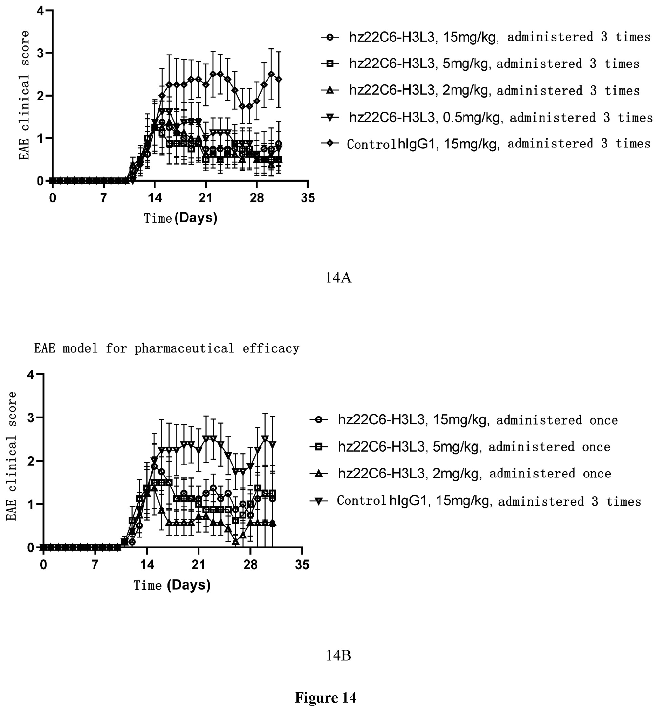

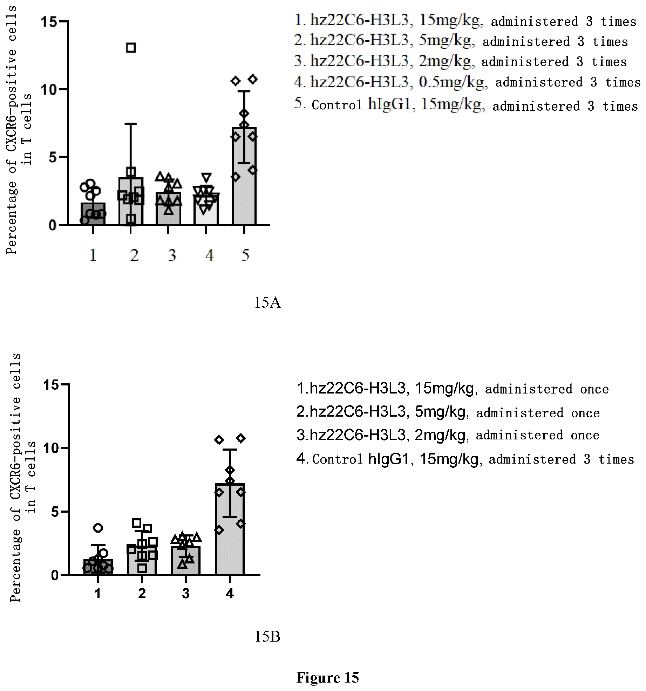

Mice were grouped and administered a drug intraperitoneally when the average EAE score of respective mice reached approximately 1. The drug is anti-CXCR6 antibody hz22C6-H3L3 of the present invention, or control hIgG1 (irrelevant antibody). Multiple dose groups (15 mg/kg, 5 mg/kg, 2 mg/kg, 0.5 mg/kg) with different administration frequencies were tested for hz22C6-H3L3, with 8 mice per group. The results showed that significant alleviation of EAE symptoms was observed in all hz22C6-H3L3 groups compared with the control group, demonstrating a significant therapeutic effect. Meanwhile, multiple administrations (Figure 14A) exhibited a slightly better therapeutic efficacy than single administration (Figure 14B), and the therapeutic efficacies of multiple administrations at different doses were comparable. Flow cytometric analysis of blood samples collected on day 24 post-modeling revealed a high proportion of CXCR6-positive T cells (CXCR6⁺ T cells) in the peripheral blood of control mice, whereas the proportion of CXCR6⁺ T cells in the peripheral blood of each treatment group was significantly reduced (Figures 15A and 15B). This indicated that the anti-CXCR6 antibody hz22C6-H3L3 of the present invention could effectively deplete CXCR6-positive T cells in mice (without being bound by any theory, it was speculated that this occurred via a mechanism such as ADCC), thereby improving clinical symptoms and achieving therapeutic effects.

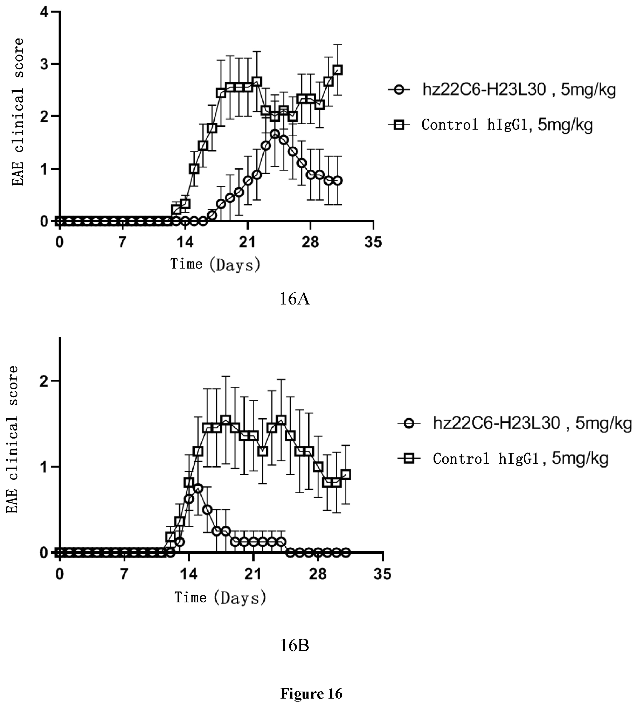

Further, the anti-CXCR6 antibody hz22C6-H23L30 of the present invention was tested for its prophylactic and therapeutic effects in the EAE model, with 8 mice per group. The results were shown in Figure 16. The results indicated that at a dose of 5 mg/kg, three prophylactic administrations (on days 4, 7, and 10 post-modeling) significantly delayed the onset of EAE in mice, while also reducing the duration and severity of EAE (Figure 16A); four therapeutic administrations (on days 14, 18, 22, and 26 post-modeling) nearly achieved complete remission of EAE symptoms in mice, demonstrating good therapeutic efficacy (Figure 16B).

The above evidences suggested the therapeutic potential of the anti-CXCR6 antibody in inflammatory diseases of the central nervous system (e.g., multiple sclerosis, MOG-antibody-associated diseases, etc.).

### 7.2 Pharmaceutical efficacy in rheumatoid arthritis model (CIA model)

The mouse CIA model is a commonly used model of rheumatoid arthritis. The mice used for modeling were also huCXCR6-knock in-C57BL/6J mice. The modeling procedure was briefly described as follows: 100 µL of CII emulsion was subcutaneously injected at the base of the mouse tail on Day 0 and Day 21, respectively (the emulsion was prepared by thoroughly mixing and emulsifying with equal volumes of 4 mg/mL chicken type-II collagen (dissolved in 50 mM acetic acid solution) and complete Freund's adjuvant comprising 4 mg/mL *Mycobacterium tuberculosis*)*.*

The clinical scoring criteria for CIA were as follows: each paw was scored independently;
Score 0: Normal;
Score 1: Erythema and swelling of any single joint (ankle joint, tarsometatarsal joint, toe joint, the same applies hereinafter);
Score 2: Erythema and swelling of any two joints;
Score 3: Erythema and swelling of all three types of joints;
Score 4: Complete erythema and swelling of the entire paw.

The pathological scoring criteria for CIA were shown in Table 7.

**Table 7. Pathological scoring criteria for the CIA model**

| Joint Lesions | Severity of Lesions | Score |
|---|---|---|
| Synovial hyperplasia with inflammatory cell infiltration in cartilage | Mild | 1.0 |
| | Severe | 2.0 |
| Pannus | Mild | 3.0 |
| | Severe | 4.0 |
| Disappearance of peri-articular space, adhesion between bone tissue and surrounding connective tissue, partial destruction of bone tissue structure | Mild | 5.0 |
| | Severe | 6.0 |
| Partial disappearance of joint space, partial destruction and fusion of bone tissue | Mild | 7.0 |
| | Severe | 8.0 |
| Fibrosis of ankle joint | | 9.0 |
| Inflammation involving phalangeal joints | | +1.0* |
| Inflammation involving bone marrow | | +1.0* |

| | | |
|---|---|---|
| *: +1.0 indicated an additional 1.0 point added to the existing score. | | |

CIA clinical scoring of mice began on Day 21. Mice with clinical CIA symptoms were evenly divided into two groups according to their scores and administered intraperitoneally with the drug (hz22C6-H23L30 or control hIgG1, n=12). The dose was 10 mg/kg, administered once every three days for seven times.

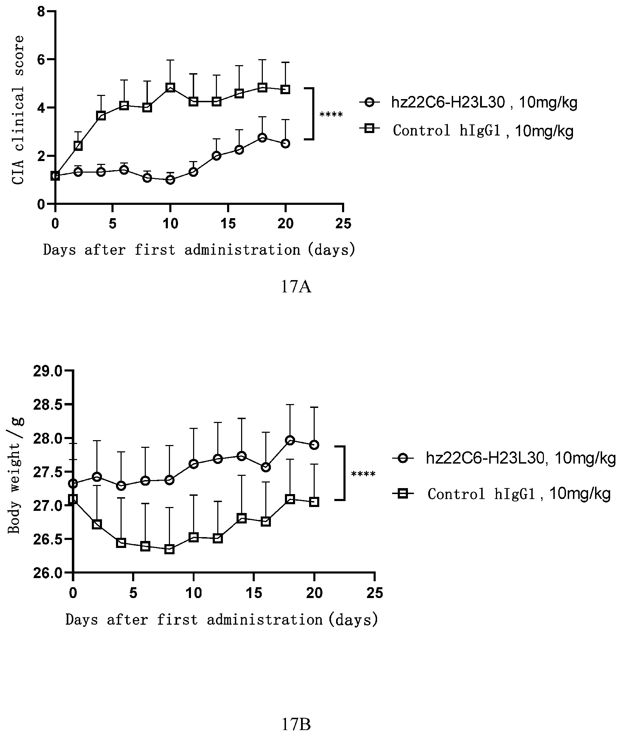

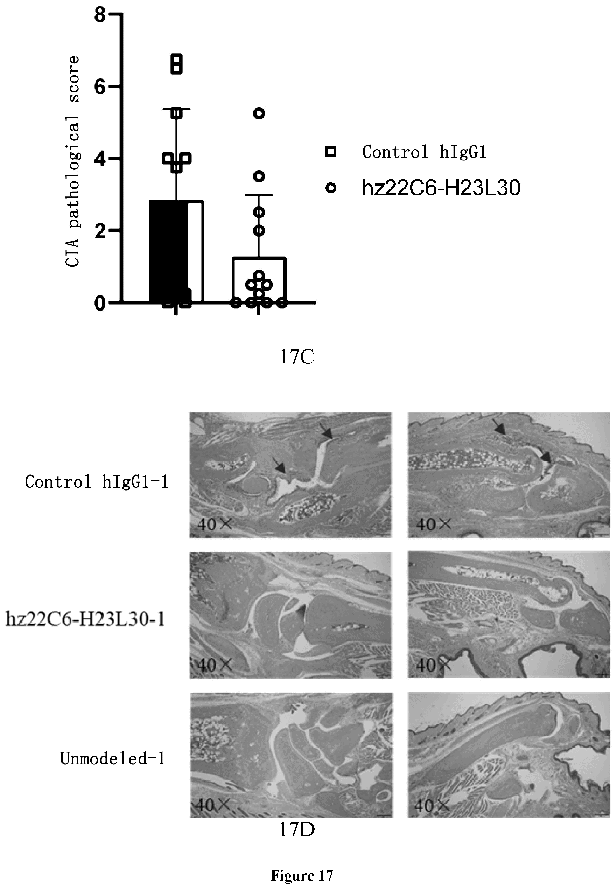

The results were shown in Figure 17. The results indicated that after administration of the anti-CXCR6 antibody hz22C6-H23L30 of the present invention, the clinical symptom scores for CIA in mice was significantly improved (Figure 17A). Mice in the hz22C6-H23L30 group exhibited significantly higher body weight than those in the control hIgG1 group (Figure 17B). Meanwhile, the pathological score of the hz22C6-H23L30 group was lower than that of the control hIgG1 group (Figures 17C and 17D, showing results from the first mouse). These results demonstrated the therapeutic potential of the anti-CXCR6 antibody in rheumatoid arthritis.

### 7.3 Pharmaceutical efficacy in mouse graft-versus-host disease model (GvHD model)

The model was established by inoculating NCG mice (6-8 weeks old, female) via tail vein with screened human PBMCs (Shanghai Schbio Biotechnology Co., Ltd., Catalog No. PBMNC100C) at a dose of 1×10⁷ cells per 200 µL. Fourteen days after the inoculation, the mice were randomly divided into two groups based on body weight and administered intraperitoneally with the drug (hz22C6-H23L30 or control hIgG1). The dose was 10 mg/kg, administered once every three days for eight times.

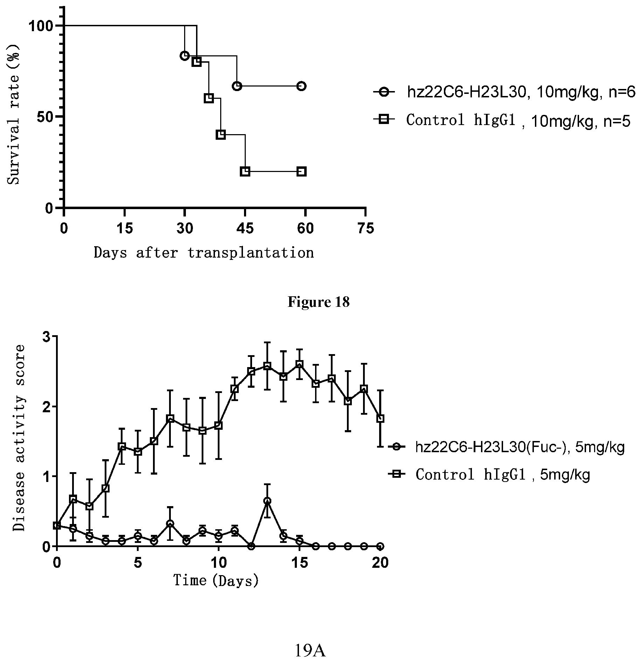

The results were shown in Figure 18. The results indicated that after administration of the anti-CXCR6 antibody hz22C6-H23L30 of the present invention, the survival rate of GvHD model mice was improved, suggesting that the anti-CXCR6 antibody had therapeutic potential in GvHD.

### 7.4 Pharmaceutical efficacy in inflammatory bowel disease model (IBD model)

A CD4⁺CD25⁻ T-cell transfer IBD model was used to evaluate the efficacy of the anti-CXCR6 antibody of the present invention. The modeling procedure was briefly described as follows: Donor mice were huCXCR6-knock in-C57BL/6J transgenic mice, and recipient mice were B6-RAG^{-/-} mice (GemPharmatech Co., Ltd.). Splenocytes from donor mice were sorted using CD4⁺CD25⁻ magnetic beads, and the sorted cells were injected into recipient mice via tail vein at a dose of 5E6 cells per mouse. Following injection, disease activity scores were assessed at regular intervals (twice a week before grouping, and once daily after grouping) based on three indicators: body weight, fecal consistency, and fecal occult blood. The scoring criteria were shown in Table 8:

**Table 8. IBD disease activity scoring scale**

| Score | Body weight Loss (%) | Fecal consistency | Fecal occult blood |
|---|---|---|---|
| 0 | - | Normal | Negative (-) |
| 1 | 1-5 | Loose but non-adherent to perianal area | Positive (±) |
| 2 | 5-10 | Loose and adherent to perianal area | Positive (+) |
| 3 | 10-15 | Diarrhea | Positive (++) |
| 4 | >15 | Bloody loose stool | Grossly bloody stool (>++) |

| | | | |
|---|---|---|---|
| Disease activity score for mice = (body weight loss score + fecal consistency score + fecal occult blood score) / 3. | | | |

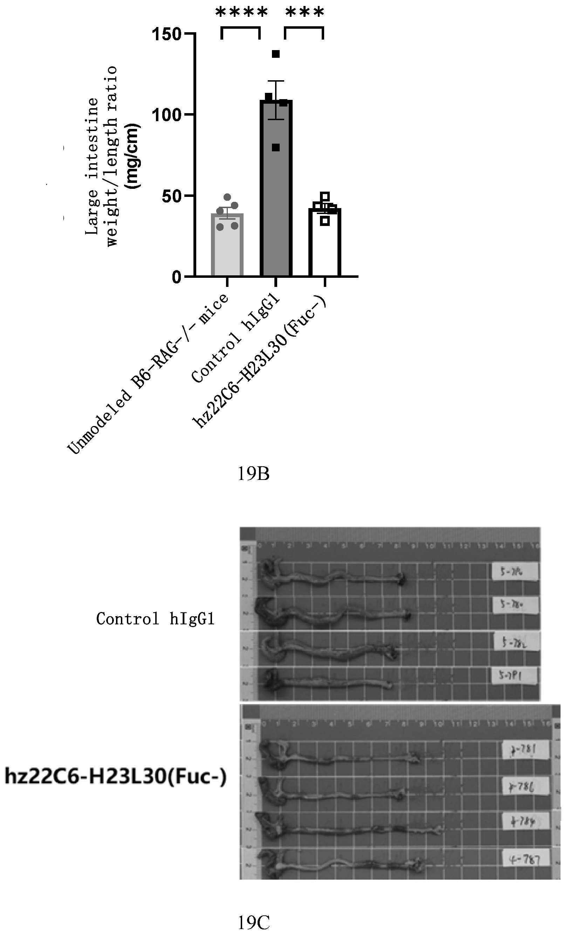

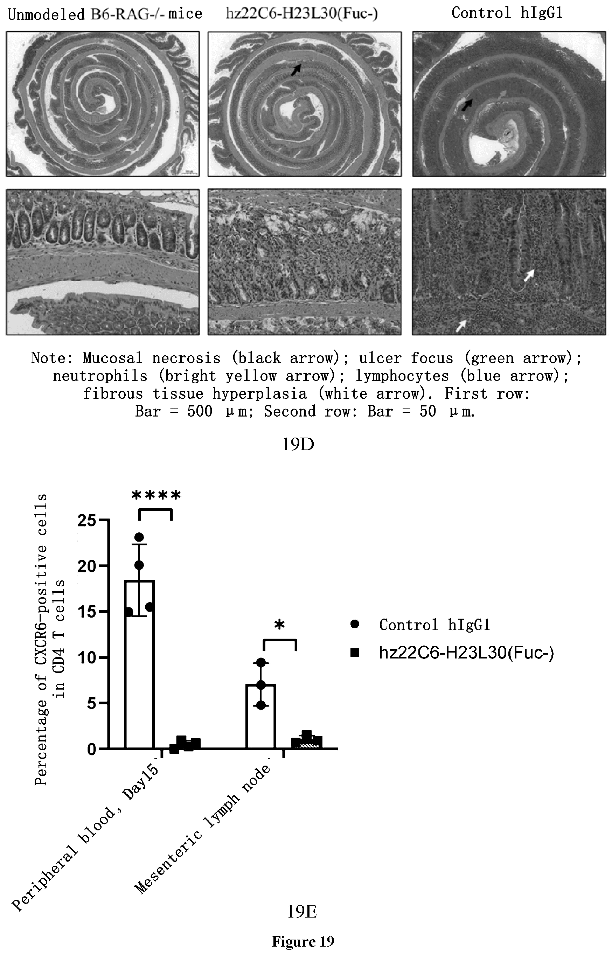

When the average disease activity score of all mice reached approximately 0.3, the mice were grouped and administered with the drug (n = 4; the day of grouping was designated as D0), hz22C6-H23L30 (Fuc⁻) or the control hIgG1 antibody, at a dose of 5 mg/kg via intraperitoneal injection twice weekly. The results were shown in Figure 19. The results indicated that following administration of the anti-CXCR6 antibody of the present invention, the clinical symptoms of IBD in mice were significantly improved, the disease activity score was significantly reduced, and complete remission was achieved after D16 (Figure 19A). On D20 of the experimental endpoint, tissue samples were collected. The large intestine weight-to-length ratio (a higher ratio indicated a more severe degree of IBD) in the hz22C6-H23L30 (Fuc-) group was comparable to that of unmodeled B6 RAG-/- mice and significantly lower than that of the control hIgG1 group (Figures 19B and 19C). HE pathological results revealed that the large intestines of mice in the control hIgG1 group exhibited extensive fibrous tissue hyperplasia, neutrophil and lymphocyte infiltration, and foci of mucosal necrosis and ulceration. These pathological features were either mild or completely absent in the hz22C6-H23L30 (Fuc-) group (Figure 19D). Meanwhile, peripheral blood was collected on D15 and mesenteric lymph nodes were harvested at the experimental endpoint for flow cytometric analysis. The results showed that the proportion of CXCR6⁺CD4⁺ T cells in peripheral blood and mesenteric lymph nodes was high in the control hIgG1 group, whereas the CXCR6⁺CD4⁺ T cell population was significantly depleted in the hz22C6-H23L30 (Fuc⁻) group (Figure 19E). Taken together, these results demonstrated the critical role of CXCR6 in the pathogenesis of IBD model, and suggested that anti-CXCR6 antibodies had therapeutic potential in inflammatory bowel disease.

### 7.5 Pharmaceutical efficacy in a vitiligo model

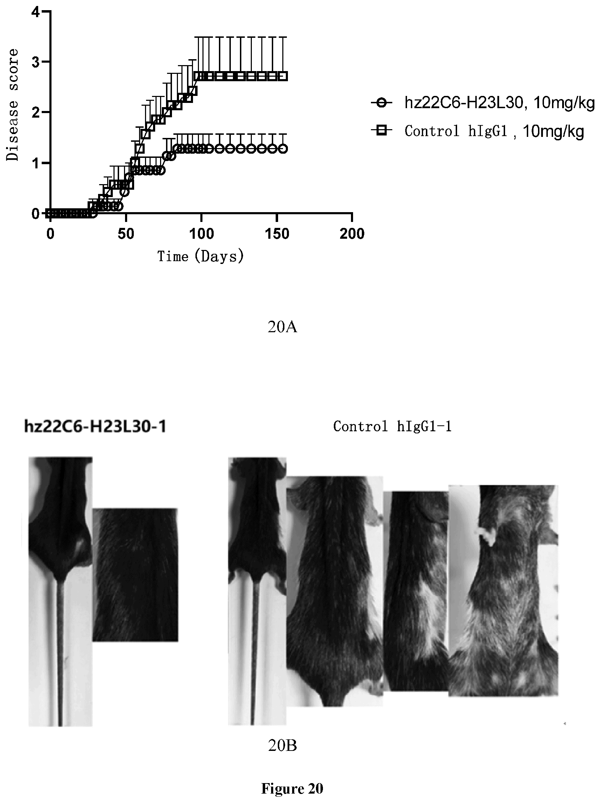

A mouse vitiligo model was used to evaluate the therapeutic efficacy of the anti-CXCR6 antibody of the present invention. The modeling procedure was briefly described as follows: On Day 0 (D0), 2E5 B16F10 melanoma cells were subcutaneously inoculated into the back of huCXCR6-knock in-C57BL/6J transgenic mice; on Day 4 (D4) and Day 10 (D10), two injections of 200 µg anti-mouse CD4 antibody (BioXcell, Catalog NO. BE0003-3-50MG) were administered; Around D21, the subcutaneous tumors were resected, and the mice were randomly divided into groups. Each group (n=7) was administered hz22C6-H23L30 or the control hIgG1 antibody at a dose of 10 mg/kg via intraperitoneal injection twice weekly. The progression of vitiligo in the mice was continuously monitored and scored from 0 to 7 according to the area of white fur. The results indicated that administration of the anti-CXCR6 antibody of the present invention alleviated the progression of vitiligo in the mice (Figures 20A and 20B), suggesting that antibodies targeting CXCR6 had therapeutic potential in vitiligo.

### Example 8: Pharmaceutical efficacy of anti-CXCR6 antibody in type 1 diabetes (T1D)

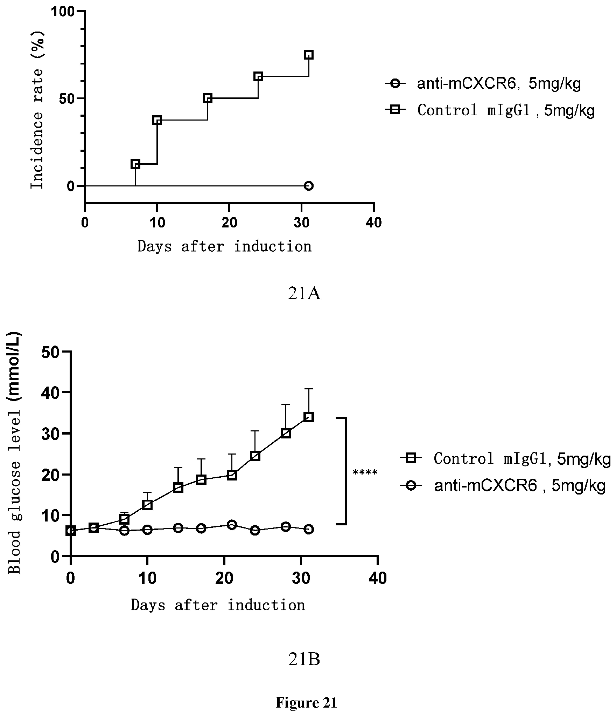

Female NOD mice develop T1D spontaneously, but the onset of the disease is delayed. Therefore, an accelerated T1D model in NOD mice was used to evaluate the therapeutic efficacy of the anti-CXCR6 antibody. The modeling procedure was briefly described as follows: On Day 0 (D0) and Day 14 (D14), NOD mice (GemPharmatech Co., Ltd.) were injected with 200 µg of anti-mouse PD-1 antibody to induce T1D onset. Meanwhile, starting from D0, mice in each group (n=7 or 8) were intraperitoneally injected with a custom-made, defucosylated anti-mouse CXCR6 antibody or control mIgG antibody at a dose of 5 mg/kg twice weekly. Blood glucose levels in mice were continuously monitored starting from D0 (twice weekly) to observe the onset of T1D in the mice. T1D onset criteria: fasting blood glucose concentration ≥ 13.9 mmol/L was defined as T1D onset. The results were shown in Figure 21. The results indicated that the anti-mouse CXCR6 antibody completely inhibited the onset of T1D (Figure 21A) and maintained normal blood glucose levels in the mice (Figure 21B). This Example suggests that antibodies targeting CXCR6 had therapeutic potential in T1D.

The foregoing description of the specific embodiments of the present invention does not limit the present invention. Those skilled in the art may make various modifications or variations based on the present invention, all of which shall fall within the scope of the appended claims of the present invention, as long as they do not depart from the spirit of the present invention.

## Claims

1. An anti-CXCR6 antibody or an antigen-binding fragment thereof, wherein the antibody or antigen-binding fragment thereof comprises heavy chain CDRs, namely heavy chain CDR1 (H-CDR1), heavy chain CDR2 (H-CDR2), and heavy chain CDR3 (H-CDR3), and light chain CDRs, namely light chain CDR1 (L-CDR1), light chain CDR2 (L-CDR2), and light chain CDR3 (L-CDR3); wherein the heavy chain CDRs and light chain CDRs are as follows:
(1) Sequentially comprising H-CDR1, H-CDR2, and H-CDR3 with the amino acid sequences shown as SEQ ID NO: 7, SEQ ID NO: 8, and SEQ ID NO: 9; and sequentially comprising L-CDR1, L-CDR2, and L-CDR3 with the amino acid sequences shown as SEQ ID NO: 11, SEQ ID NO: 12, and SEQ ID NO: 13;
(2) Sequentially comprising H-CDR1, H-CDR2, and H-CDR3 with the amino acid sequences shown as SEQ ID NO: 7, SEQ ID NO: 10, and SEQ ID NO: 9; and sequentially comprising L-CDR1, L-CDR2, and L-CDR3 with the amino acid sequences shown as SEQ ID NO: 11, SEQ ID NO: 12, and SEQ ID NO: 14; or
(3) Sequentially comprising H-CDR1, H-CDR2, and H-CDR3 with the amino acid sequences shown as SEQ ID NO: 7, SEQ ID NO: 8, and SEQ ID NO: 9; and sequentially comprising L-CDR1, L-CDR2, and L-CDR3 with the amino acid sequences shown as SEQ ID NO: 15, SEQ ID NO: 16, and SEQ ID NO: 14.

2. The antibody or antigen-binding fragment thereof according to claim 1, **characterized in that** the antibody or antigen-binding fragment thereof specifically targets CXCR6, preferably human CXCR6;
Optionally, the antibody or antigen-binding fragment thereof possesses cross-species binding activity to human and monkey CXCR6.

3. The antibody or antigen-binding fragment thereof according to claim 1 or 2, **characterized in that** the antibody or antigen-binding fragment thereof comprises a heavy chain variable region and a light chain variable region, and the heavy chain variable region and the light chain variable region comprise one of the amino acid sequence combinations selected from the group consisting of:
(1) the amino acid sequence shown as SEQ ID NO: 1, or an amino acid sequence having at least 75% identity thereto; and the amino acid sequence shown as SEQ ID NO: 4, or an amino acid sequence having at least 75% identity thereto;
(2) the amino acid sequence shown as SEQ ID NO: 2, or an amino acid sequence having at least 75% identity thereto; and the amino acid sequence shown as SEQ ID NO: 5, or an amino acid sequence having at least 75% identity thereto; or
(3) the amino acid sequence shown as SEQ ID NO: 3, or an amino acid sequence having at least 75% identity thereto; and the amino acid sequence shown as SEQ ID NO: 6, or an amino acid sequence having at least 75% identity thereto.

4. The antibody or antigen-binding fragment thereof according to any one of claims 1 to 3, **characterized in that** the antibody is a murine antibody, a rabbit antibody, a human antibody, or a murine-derived antibody, a chimeric antibody, a fully or partially humanized antibody, or a derivatized antibody;
Or, the antigen-binding fragment of the antibody is in any form of antibody fragment selected from scFv, BsFv, dsFv, (dsFv)₂, Fab, Fab', F(ab')₂ and Fv;
Optionally, the antibody or antigen-binding fragment thereof further comprises a heavy chain constant region (CH) and/or a light chain constant region (CL), preferably a human or murine heavy chain constant region and/or light chain constant region; preferably, the antibody or fragment thereof comprises a heavy chain constant region of IgG, IgA, IgM, IgD, or IgE and/or a light chain constant region of κ-type or λ-type.

5. The antibody or antigen-binding fragment thereof according to any one of claims 1 to 4, **characterized in that** the antibody is a monoclonal antibody; preferably, the antibody is an immunoglobulin, for example, the immunoglobulin is of human IgA, IgD, IgE, IgG, or IgM class; preferably, the antibody is of human IgG1 or IgG4 subclass;
Preferably, the antibody or antigen-binding fragment thereof exhibits antibody-dependent cell-mediated cytotoxicity (ADCC), and preferably has enhanced ADCC activity.

6. A nucleic acid molecule, comprising one encoding the antibody or antigen-binding fragment thereof according to any one of claims 1 to 5.

7. A vector, comprising the nucleic acid molecule according to claim 6.

8. A host cell, comprising the nucleic acid molecule according to claim 6 or the vector according to claim 7.

9. A composition, comprising the antibody or antigen-binding fragment thereof according to any one of claims 1 to 5, the nucleic acid molecule according to claim 6, the vector according to claim 7, or the host cell according to claim 8, and optionally a pharmaceutically acceptable excipient.

10. Use of the antibody or antigen-binding fragment thereof according to any one of claims 1 to 5, the nucleic acid molecule according to claim 6, the vector according to claim 7, the host cell according to claim 8, or the composition according to claim 9 in the manufacture of a medicament for preventing, treating and/or ameliorating a disease or disorder.

11. A method for preventing, treating and/or ameliorating a disease or disorder, comprising administering to a subject in need thereof the antibody or antigen-binding fragment thereof according to any one of claims 1 to 5, the nucleic acid molecule according to claim 6, the vector according to claim 7, the host cell according to claim 8, or the composition according to claim 9.

12. Use of the antibody or antigen-binding fragment thereof according to any one of claims 1 to 5, the nucleic acid molecule according to claim 6, the vector according to claim 7, the host cell according to claim 8, or the composition according to claim 9 in the manufacture of a reagent for diagnosing a disease or disorder.

13. A method for diagnosing a disease or disorder, comprising contacting the antibody or antigen-binding fragment thereof according to any one of claims 1 to 5, the nucleic acid molecule according to claim 6, the vector according to claim 7, the host cell according to claim 8, or the composition according to claim 9 with a biological sample from a subject.

14. The use or method according to any one of claims 10 to 13, **characterized in that** the disease or disorder is associated with CXCR6 expression (including overexpression);
Preferably, the disease or disorder is acute graft-versus-host disease, chronic graft-versus-host disease, autoimmune hepatitis, Crohn's disease, ulcerative colitis, contact dermatitis, rheumatoid arthritis, psoriasis, psoriatic arthritis, juvenile rheumatoid arthritis, multiple sclerosis, non-alcoholic steatohepatitis, systemic lupus erythematosus, uveitis, type I diabetes, ankylosing spondylitis, myelin oligodendrocyte glycoprotein-associated disease, Graves' disease, Sjögren's syndrome;
Preferably, the subject is a mammal; more preferably, the subject is a human.

15. A kit, comprising the antibody or antigen-binding fragment thereof according to any one of claims 1 to 5, the nucleic acid molecule according to claim 6, the vector according to claim 7, the host cell according to claim 8, or the composition according to claim 9.
